(19) Europäisches Patentamt
European Patent Office
Office européen des brevets

(11) EP 3 533 788 A1

(12) EUROPEAN PATENT APPLICATION

(43) Date of publication:
**04.09.2019 Bulletin 2019/36**

(21) Application number: **18159060.5**

(22) Date of filing: **28.02.2018**

(51) Int Cl.:
*C07D 239/26* (2006.01) *C07F 9/53* (2006.01)
*C07D 417/14* (2006.01) *C07D 471/04* (2006.01)
*C07D 263/57* (2006.01) *C07D 271/107* (2006.01)
*C07D 277/22* (2006.01) *C07D 277/66* (2006.01)
*C07D 513/04* (2006.01) *C07D 285/12* (2006.01)
*C07F 9/6512* (2006.01) *C07F 9/653* (2006.01)
*C07F 9/6539* (2006.01) *C07F 9/6541* (2006.01)
*C07F 9/6561* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Novaled GmbH**
**01307 Dresden (DE)**

(72) Inventors:
- **SCHULZE, Benjamin**
  **01099 Dresden (DE)**
- **CARDINALI, Francois**
  **01099 Dresden (DE)**
- **HUANG, Qiang**
  **01099 Dresden (DE)**

(74) Representative: **Michalski Hüttermann & Partner Patentanwälte mbB**
**Speditionstraße 21**
**40221 Düsseldorf (DE)**

# (54) ORGANIC MATERIAL FOR AN ELECTRONIC OPTOELECTRONIC DEVICE AND ELECTRONIC DEVICE COMPRISING THE ORGANIC MATERIAL

(57) The present invention relates to an organic material and to an electronic device comprising the organic material, particularly to an electroluminescent device, particularly to an organic light emitting diode (OLED), wherein the semi-conducting material comprises a double-heteroarylsubstituted aryl or heteroaryl, further substituted by a substituted aryl moiety.

Fig. 1

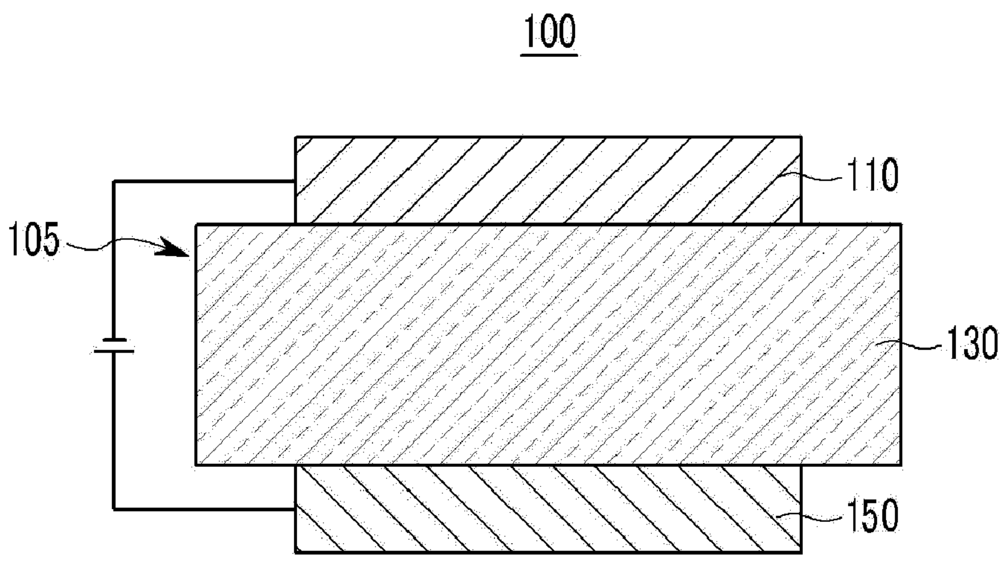


EP 3 533 788 A1

## Description

### Technical Field

The present invention relates to an organic material and to an electronic device comprising the organic material, particularly to an electroluminescent device, particularly to an organic light emitting diode (OLED); the invention pertains also to a device comprising the electric device and/or the electroluminescent device, particularly to a display device, particularly to a display device comprising the OLED.

### Background Art

Organic light-emitting diodes (OLEDs), which are self-emitting devices, have a wide viewing angle, excellent contrast, quick response, high brightness, excellent driving voltage characteristics, and color reproduction. A typical OLED includes an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. There is continuing demand for development of improved materials, with the aim that operational voltage is as low as possible while brightness/luminance is high, and that injection and flow of holes and electrons is balanced, so that an OLED having the above-described structure has excellent efficiency and/or a long lifetime.

One of well-established approaches for achieving low operational voltages, high current densities and luminance efficiencies is electrical p- and/or n-doping in charge injection/charge transport layers, and especially redox doping which generates doped layers with high charge carrier concentrations.

### DISCLOSURE

Aspects of the present invention provide an organic material for an electronic device, particularly for a light-emitting or electroluminescent device comprising an emission layer and at least two electrodes, for increasing the efficiency, such as the external quantum efficiency EQE, and for achieving low operating voltage and long lifetime, particularly in top and/or bottom emission organic light-emitting diodes (OLEDs).

Another aspect of the present invention provides an electronic device, particularly an electroluminescent device comprising the organic material. Still another aspect of the present invention provides a display device comprising the electroluminescent device.

According to an aspect of the present invention, there is provided an organic material for an electroluminescent device having the Formula (I):

$$(Het)_2 - Ar^1 - [L(Ar^2)_m(R^1)_n(R^2)_p]_q \qquad (I)$$

wherein

Het is an aryl or heteroaryl substituted or unsubstituted $C_2$ to $C_{30}$ heteroaryl group, whereby Het is not carbazoyl nor triazolyl,

$Ar^1$ is a $C_6$ to $C_{30}$ aryl group or $C_2$ to $C_{30}$ heteroaryl group, whereby $Ar^1$ is not triazine,

L is a $C_6$ to $C_{30}$ substituted or unsubstituted aryl group

$Ar^2$ is a substituted, aryl or heteroaryl or $R^1$ substituted or unsubstituted $C_6$ to $C_{42}$ aryl group or $C_2$ to $C_{42}$ heteroaryl group

$R^1$ is a polar uncharged organic moiety

$R^2$ is selected from the group comprising aryl or heteroaryl- substituted vinyl, -O-, -S-, -NR'-, alkylene, arylene.

m to p are independently an integer of 0 to 5

q is 1, 2 or 3, whereby when q > 1 then any moieties L can be different from each other and two suitable moieties L may form a macrocyclus with $Ar^1$ and $R^2$;

whereby when Het is triphenylsubstituted imidazole and L is anthracenyl or when Het is benzimidazole then $Ar^1$ is not carbazolyl

In the present specification, when a definition is not otherwise provided, "substituted" refers to one substituted with a deuterium, $C_1$ to $C_{12}$ alkyl and $C_1$ to $C_{12}$ alkoxy.

However, in the present specification "aryl or heteroaryl substituted" refers to a substitution with one or more aryl and/or heteroaryl and/or $R^1$ groups, which themselves may be substituted with one or more aryl and/or heteroaryl and/or $R^1$ groups.

In the present specification, when a definition is not otherwise provided, an "alkyl group" refers to a saturated aliphatic hydrocarbyl group. The alkyl group may be a $C_1$ to $C_{12}$ alkyl group. More specifically, the alkyl group may be a $C_1$ to $C_{10}$ alkyl group or a $C_1$ to $C_6$ alkyl group. For example, a $C_1$ to $C_4$ alkyl group includes 1 to 4 carbons in alkyl chain, and may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a t-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenylyl, and polycyclic groups comprising fused rings, like naphtyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" is understood the way that two aryl rings are considered fused when they share at least two common $sp^2$-hybridized carbon atoms

In the present specification, the single bond refers to a direct bond.

In the context of the present invention, "different" means that the compounds do not have an identical chemical structure.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electron formed in the cathode may be easily injected into the emission layer and transported in the emission layer due to conductive characteristics according to a lowest unoccupied molecular orbital (LUMO) level.

## Advantageous Effects

Surprisingly, it was found that the organic material according to the invention solves the problem underlying the present invention by enabling devices in various aspects superior over the organic electroluminescent devices known in the art, in particular with respect to voltage and/or efficiency. These parameters are important for high efficiency and thereby increased battery life of a mobile device, for example a mobile display device.

According to a further embodiment of the invention, $Ar^1$ is selected from phenyl, pyridyl, carbazolyl and fluorenyl.

Especially it is preferred if $Ar^1$ is selected from the moieties G1 to G5:

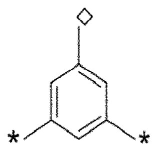
G1

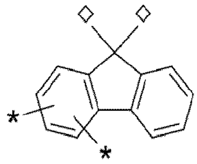
G2

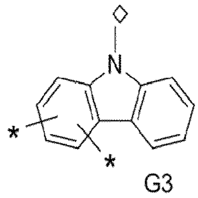
G3

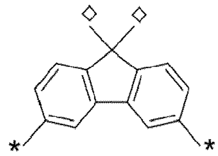
G4

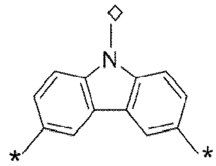
G5

Wherein
L is connected to $Ar^1$ at the positions marked with " ◊ " via single bonds; and
The two $Het^1$ are connected to $Ar^1$ at the positions marked with " * " via single bonds.
According to a further embodiment of the invention, whereby L is selected from phenyl, naphtanyl, anthracenyl and fluorenyl.
According to a further embodiment of the invention, Het is a substituted or unsubstituted bicyclic aryl moiety comprising a heteroaryl-containing five-membered ring.
According to a further embodiment of the invention, Het is phenyl substituted six-membered heteroaryl.
According to a further embodiment of the invention, Het is selected from:

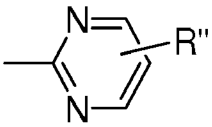
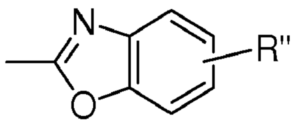
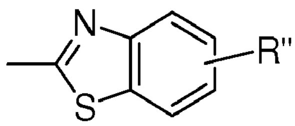
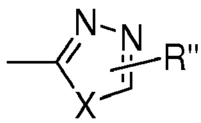

whereby X = N or S and R'' is hydrogen, phenyl, naphthyl
According to a further embodiment of the invention, the moiety Het-Rg-Het has a $C_{2v}$ - symmetry, whereby
Rg= $Ar^1$ when $Ar^1$ is monocyclic or the two moieties Het are bound to two different rings; or
Rg= the aromatic ring within $Ar^1$ to which both Het are bound
To explain: In the following Table 1 three molecules which are preferred embodiments of the present inventions are juxtaposed with their sub-moiety Het-Rg-Het:

**Table 1: Compounds with full structure and substructure Het-Rg-Het**

| Compound | Full structure | Substructure Het-Rg-Het |
|---|---|---|
| A-18 | 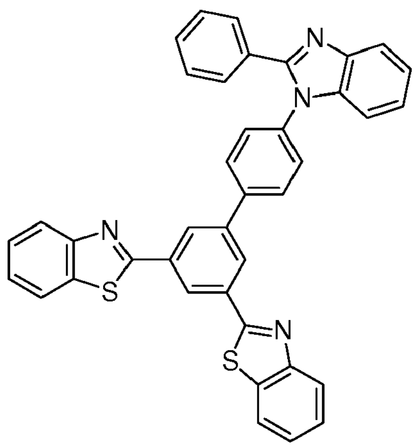 | 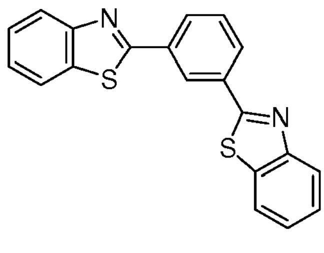 |

(continued)

| Compound | Full structure | Substructure Het-Rg-Het |
|---|---|---|
| A-19 | 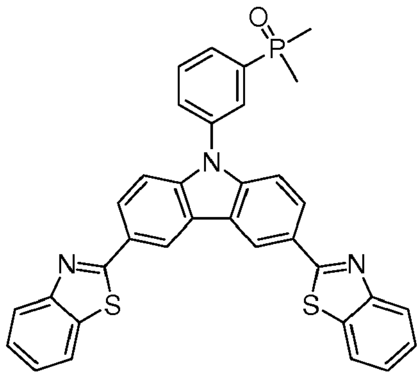 | 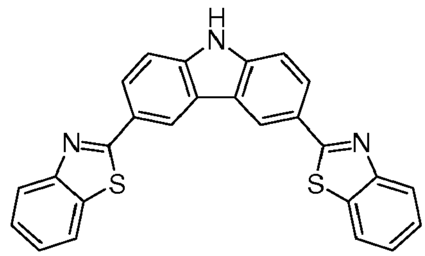 |
| A-48 | 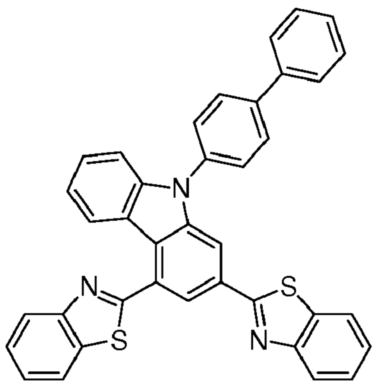 | 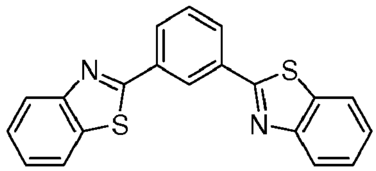 |

According to a further embodiment of the present invention, the two Het are connected to one ring within $Ar^1$ and in meta-position to each other.

According to a further embodiment of the present invention, m+n+p >0.

According to a further embodiment of the present invention, $1 \leq m+n+p \leq 5$.

According to a further embodiment of the present invention, $R^1$ is selected from substituted or unsubstituted $C_2$ to $C_{30}$ heteroaryl group, CN and phosphine oxide.

The term phosphine oxide especially means and/or includes the group -P(O) $R_1R_2$, wherein $R_1$ and $R_2$ are independently selected from: hydrogen; $C_1$-$C_6$-alkyl; phenyl; and $C_1$-$C_6$-alkyl-$C_6H_5$; and amine (to give phosphonamidate) selected from the group: -NR'$_2$, wherein each R' is independently selected from: hydrogen; $C_1$-$C_6$-alkyl; $C_1$-$C_6$-alkyl-$C_6H_5$; and phenyl, wherein when both R' are $C_1$-$C_6$-alkyl both R' together may form an -$NC_3$ to an -$NC_5$ heterocyclic ring with any remaining alkyl chain forming an alkyl substituent to the heterocyclic ring.

According to a further embodiment of the invention, $R^1$ is a moiety which is chosen so that the dipole moment of the compound $R^1$-phenyl is ≥ 0.6 Debye, preferably ≥ 0.7 Debye

The dipole moments, calculated with B3LYP_Gaussian / 6-31G*, gas phase, for compounds $R^1$-Phenyl for preferred R's are given in the following Table 2:

**Table 2: Calculated Dipole moments for selected compounds $R^1$-Phenyl**

| # | structure | dipole moment [Debye] |
|---|---|---|
| 1 | 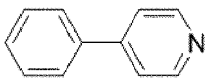 | 2,86 |
| 2 | 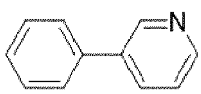 | 2,3 |

(continued)

| # | structure | dipole moment [Debye] |
|---|---|---|
| 3 | 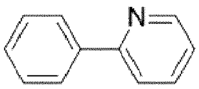 | 1,71 |
| 4 | 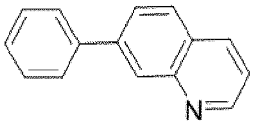 | 1,91 |
| 5 | 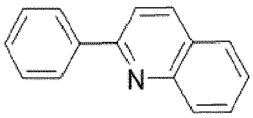 | 1,53 |
| 6 | 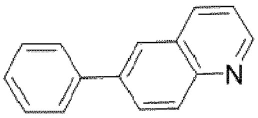 | 2,11 |
| 7 | 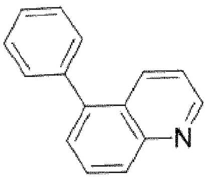 | 2,31 |
| 8 | 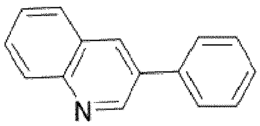 | 2,06 |
| 9 | 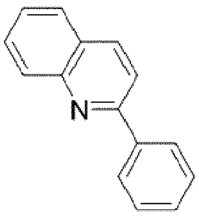 | 1,53 |
| 10 | 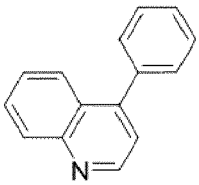 | 2,57 |
| 11 | 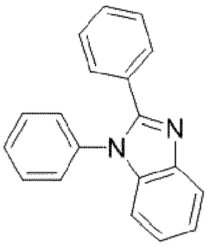 | 3,71 |

(continued)

| # | structure | dipole moment [Debye] |
|---|---|---|
| 12 | 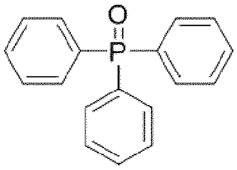 | 4,18 |
| 13 | 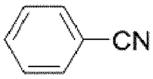 | 4,56 |
| 14 | 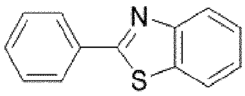 | 0,66 |
| 15 | 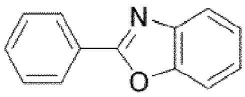 | 1,15 |
| 16 | 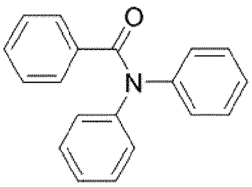 | 3,34 |
| 17 | 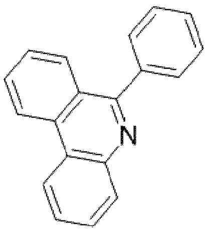 | 1,8 |
| 18 | 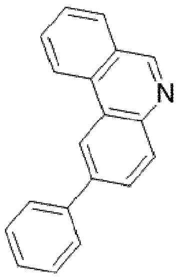 | 2,34 |
| 19 | 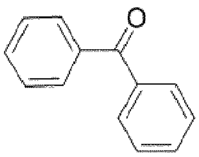 | 2,97 |

(continued)

| # | structure | dipole moment [Debye] |
|---|---|---|
| 20 | 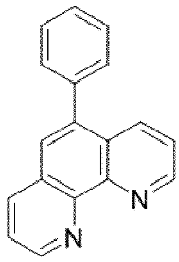 | 3,58 |
| 21 | 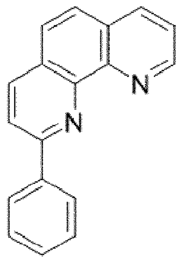 | 2,67 |
| 22 | 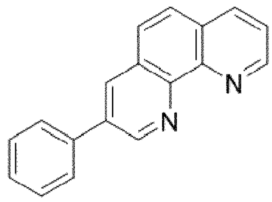 | 3,13 |
| 23 | 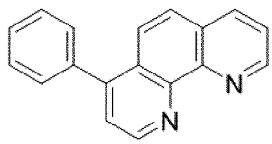 | 3,68 |
| 24 | 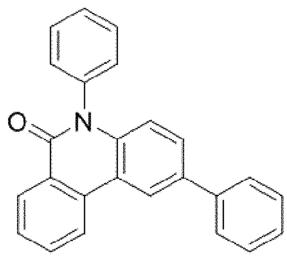 | 2,68 |

According to a further embodiment of the invention, $R^1$ is selected from

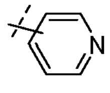 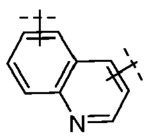 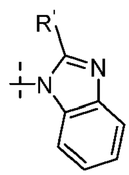 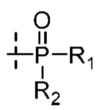 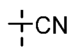

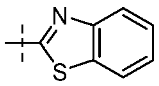
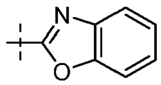
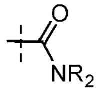
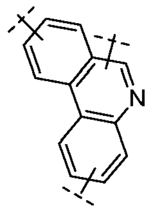
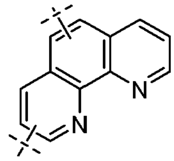

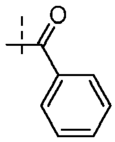
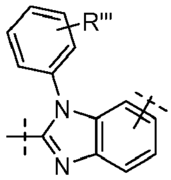
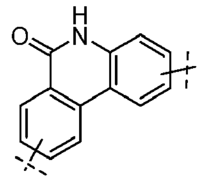

with $R_1$ and $R_2$ as defined above in the phosphine oxide definition, R’, being alkyl, cycloalkyl, aryl, heteroaryl; and R" and R"’ being independently from each other alkyl, cycloalkyl, aryl, heteroaryl.

According to a further embodiment, $R^1$ is linked to L at any of the positions indicated with an open bond crossed by a dashed line.

According to a further embodiment, the organic material has a dipole moment of ≥ 0.5 Debye.

The inventors have surprisingly found that particularly good performance can be achieved when using the organic material according to the invention in an electron transport layer in an optoelectronic device.

Additionally or alternatively the inventors have surprisingly found that particularly good performance can be achieved when using the organic material according to the invention in or as a hole blocking layer in an optoelectronic device.

The present invention furthermore relates to a electronic device comprising a first electrode, a second electrode, and arranged between the first and second electrode, a layer comprising the organic material according to the invention.

According to a further embodiment, the electronic device comprises a hole blocking layer comprising a compound according to the invention.

According to a further embodiment, the electronic device comprises an electron transport layer comprising a compound according to the invention.

According to a further embodiment, the electronic device is an electroluminescent device, preferably an organic light emitting diode.

The present invention furthermore relates to a display device comprising an electronic device according to the present invention, preferably, the display device comprises an organic light emitting diode according to the present invention.

The specific arrangements mentioned herein as preferred were found to be particularly advantageous.

Further an organic electroluminescent device having high efficiency and/or long life-span may be realized.

Hereinafter, the organic material and the device comprising it are described in more detail

Organic Material

Similar as other compounds comprised in the inventive device outside the emitting layer, the organic material may not emit light under the operation condition of an electroluminescent device, for example an OLED.

Particularly preferred may be compounds of formula I with the following structures A1 to A51.

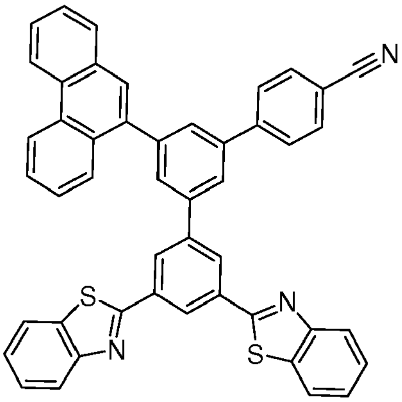

A-1

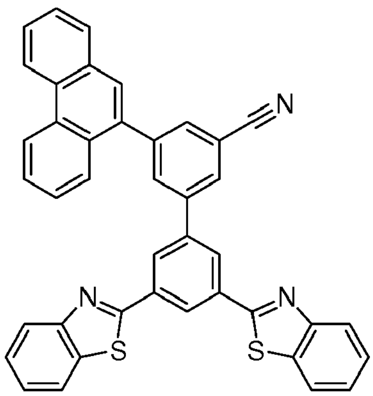

A-2

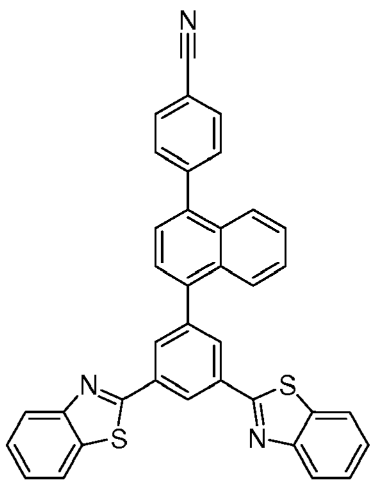

A-3

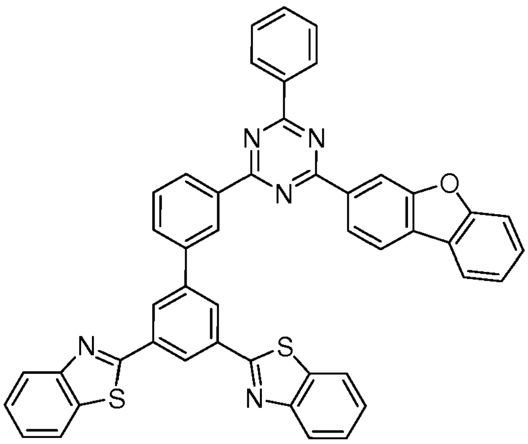

A-4

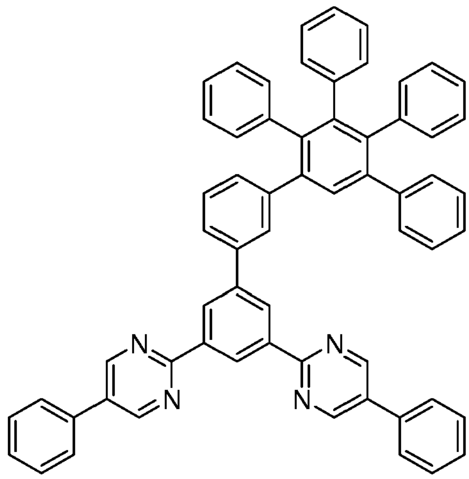

A-5

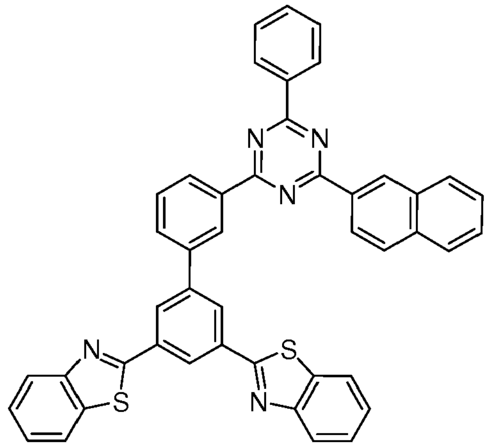

A-6

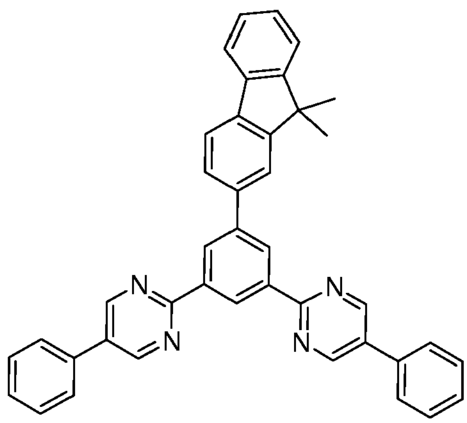

A-7

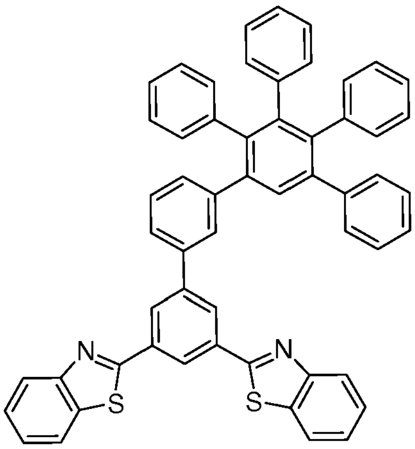
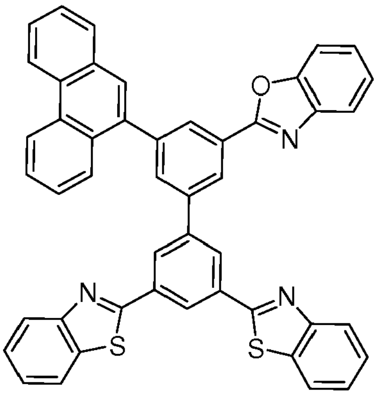
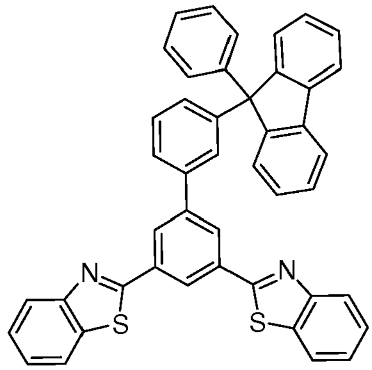

A-8 A-9 A-10

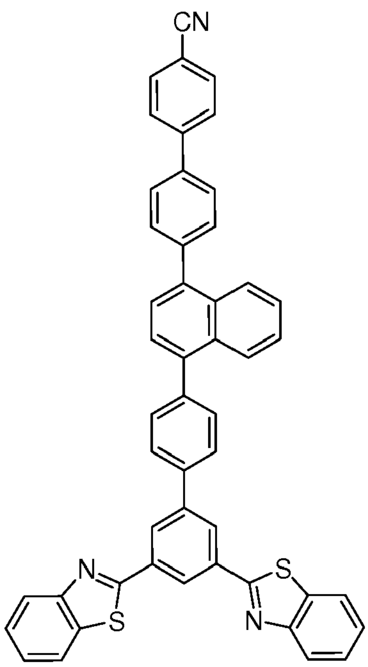
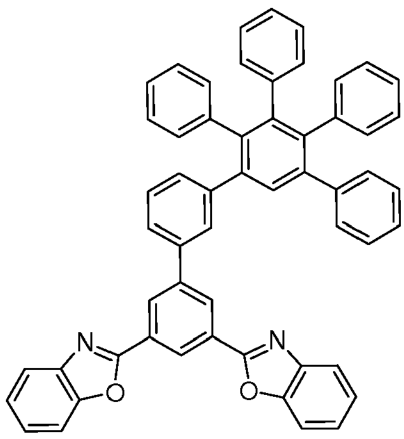
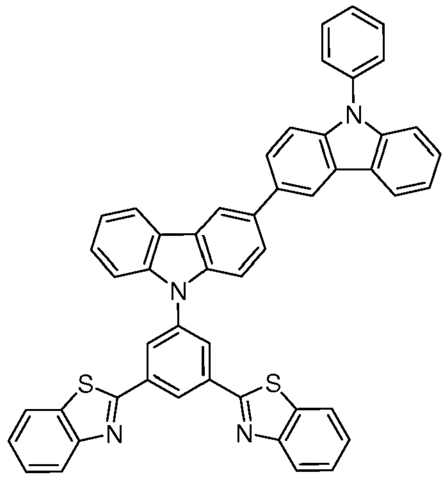

A-11 A-12 A-13

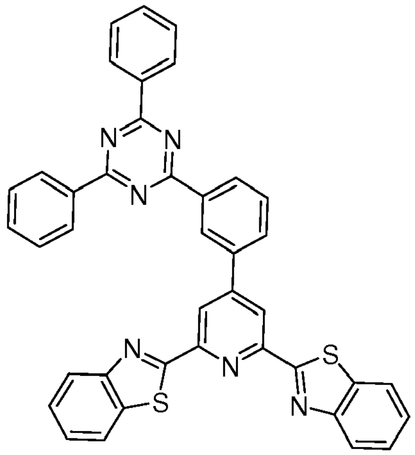

A-14

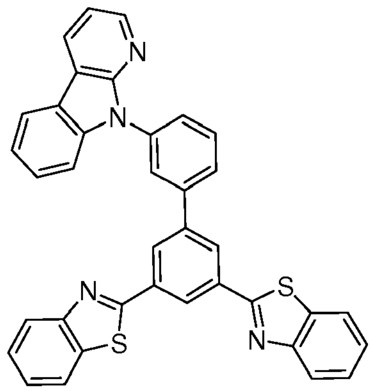

A-15

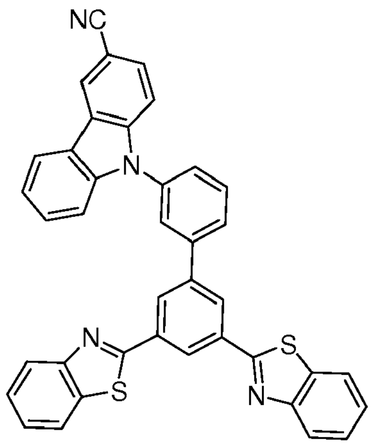

A-16

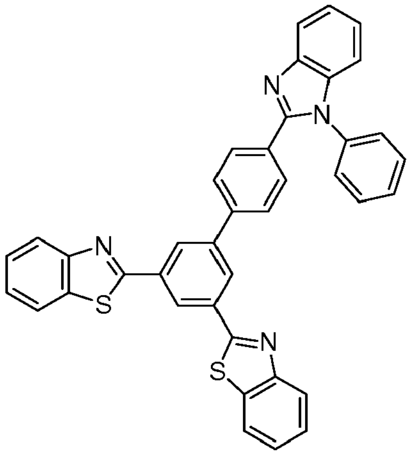

A-17

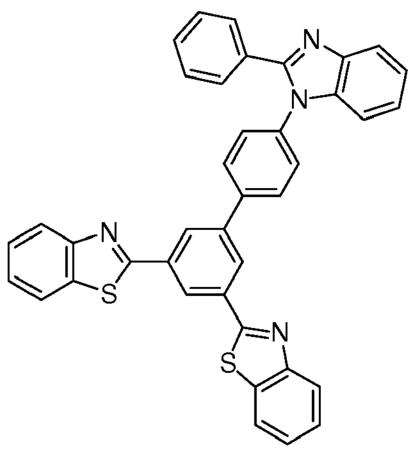

A-18

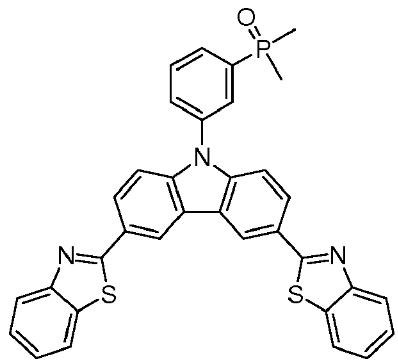

A-19

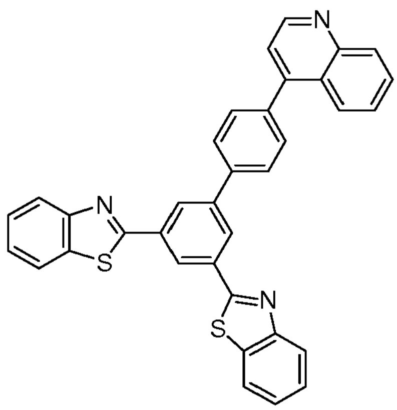

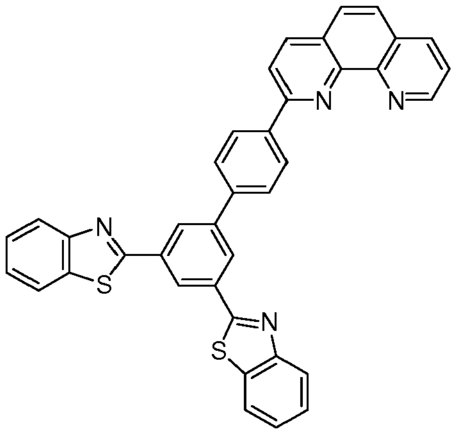

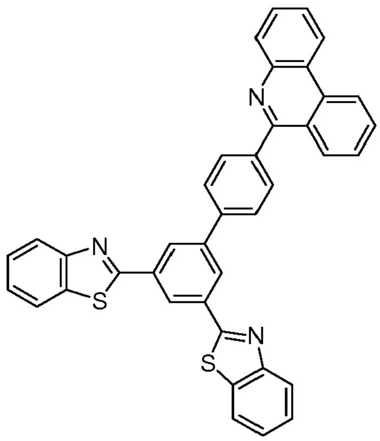

A-20 A-21 A-22

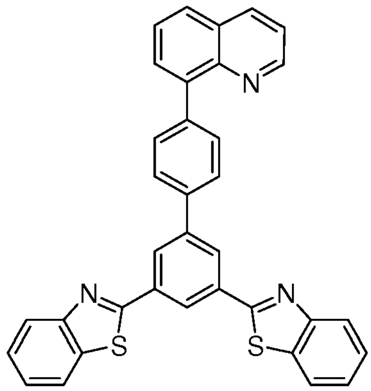

A-23

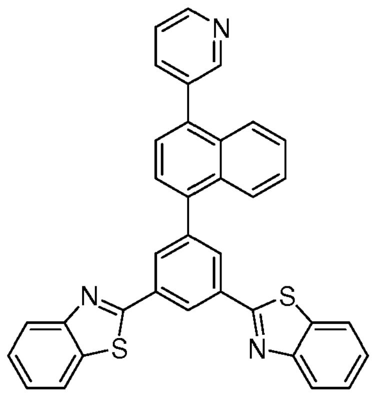

A-24

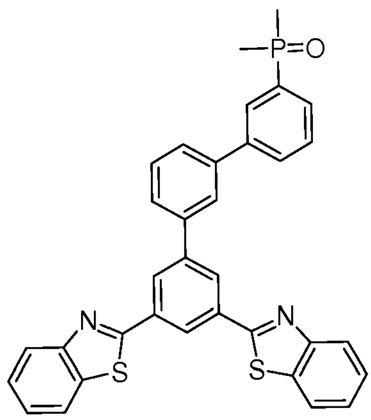

A-25

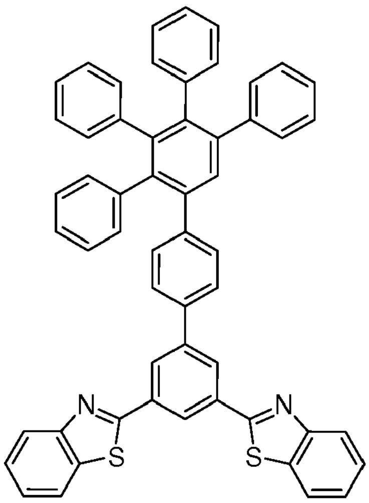

A-26

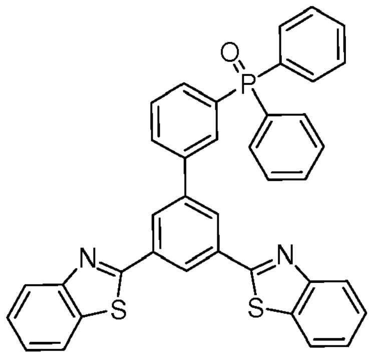

A-27

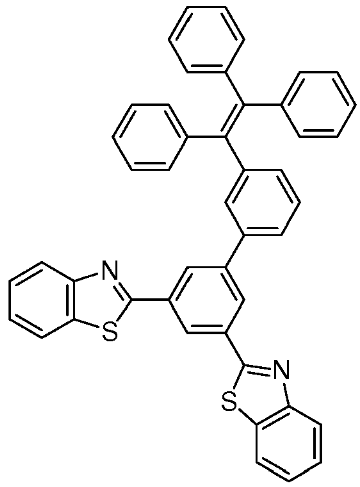

A-28

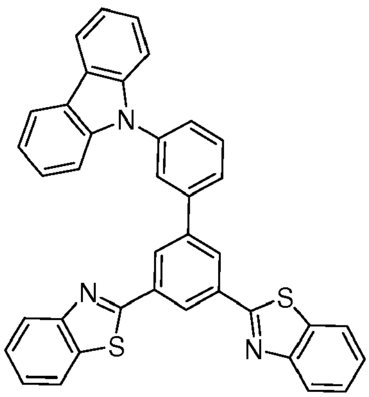

A-29

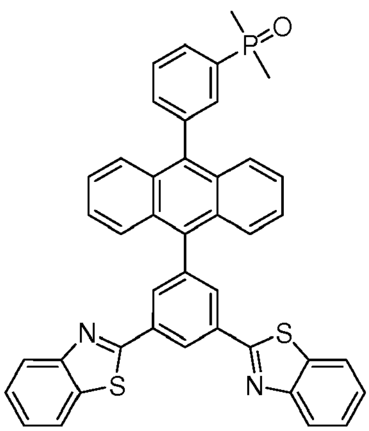

A-30

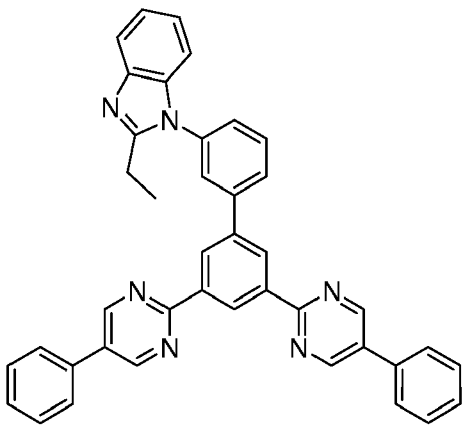

A-31

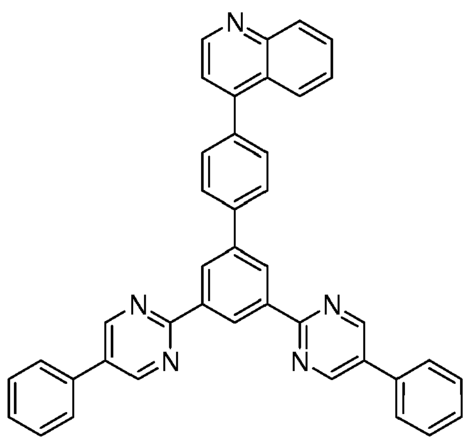

A-32

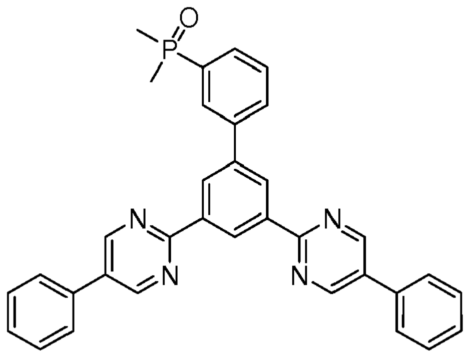

A-33

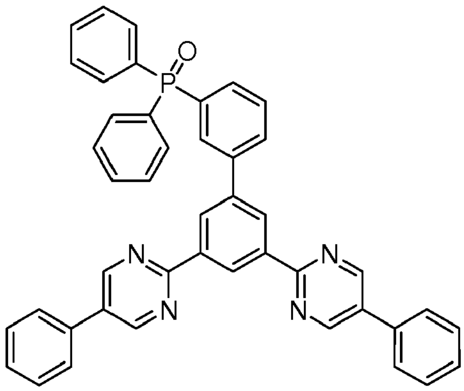

A-34

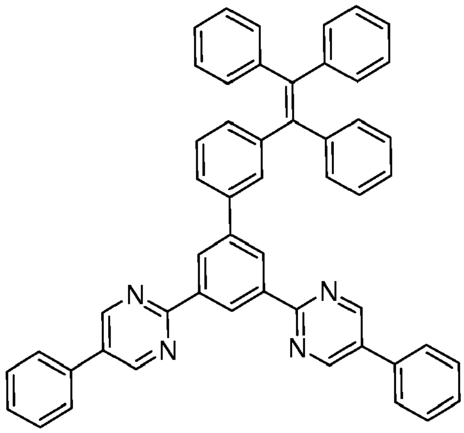

A-35

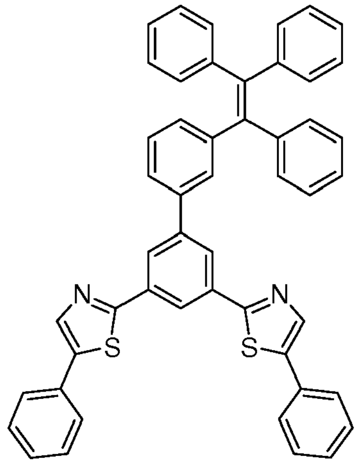

A-36

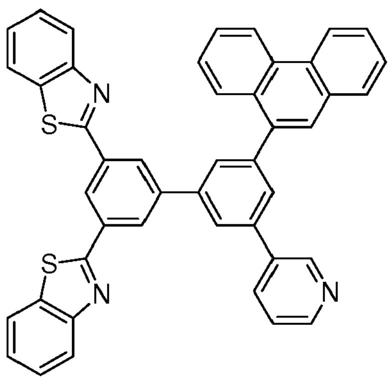

A-37

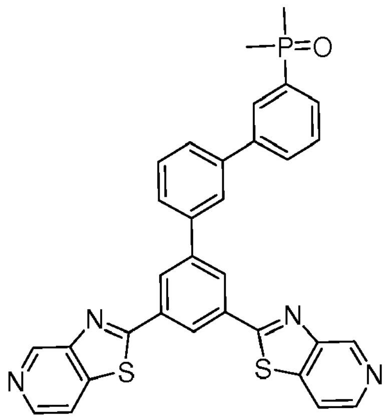

A-38

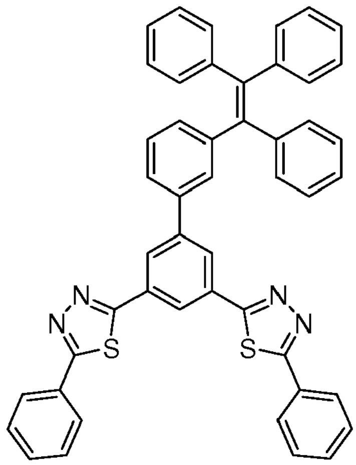

A-39

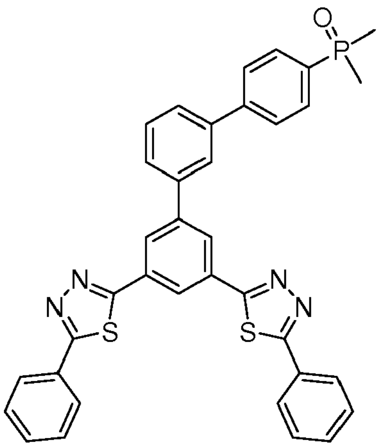

A-40

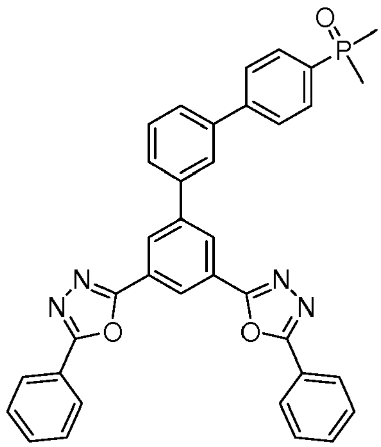

A-41

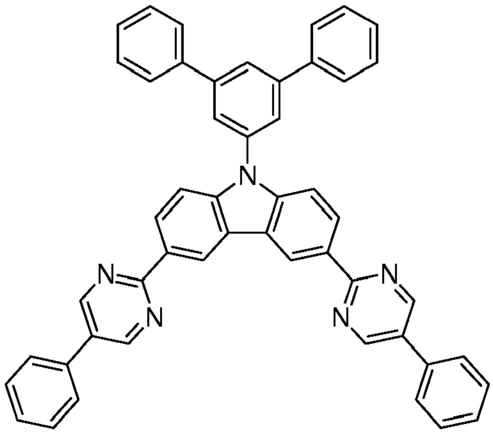
A-42

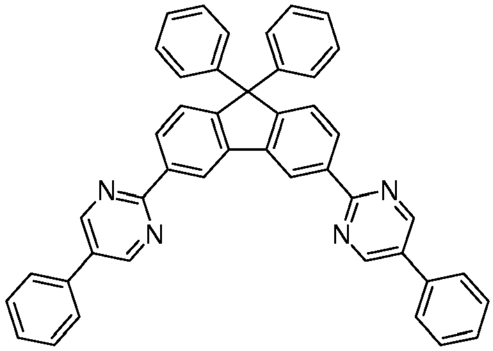
A-43

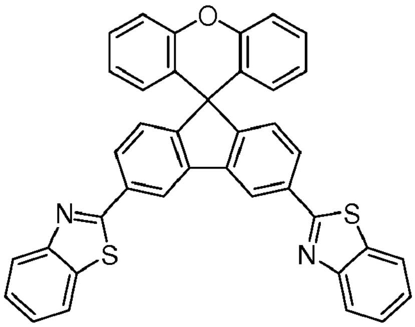
A-44

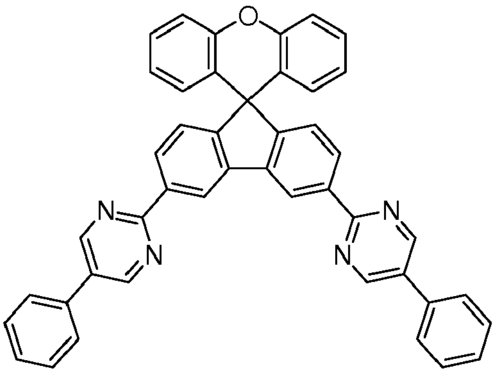
A-45

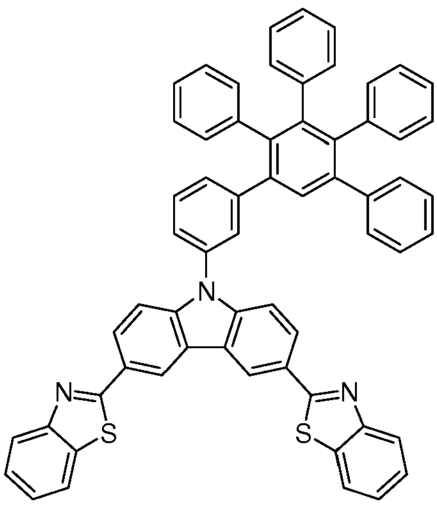
A-46

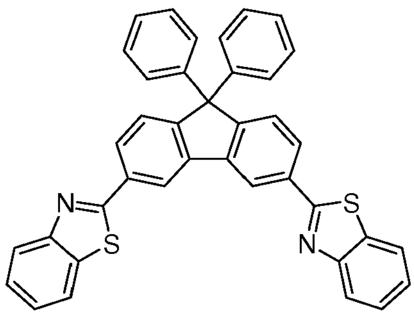
A-47

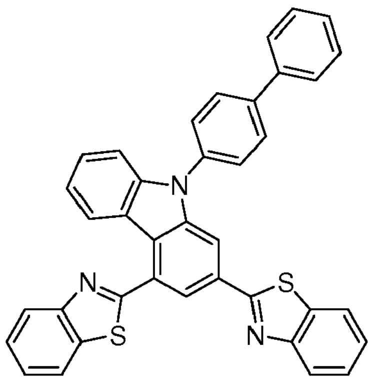

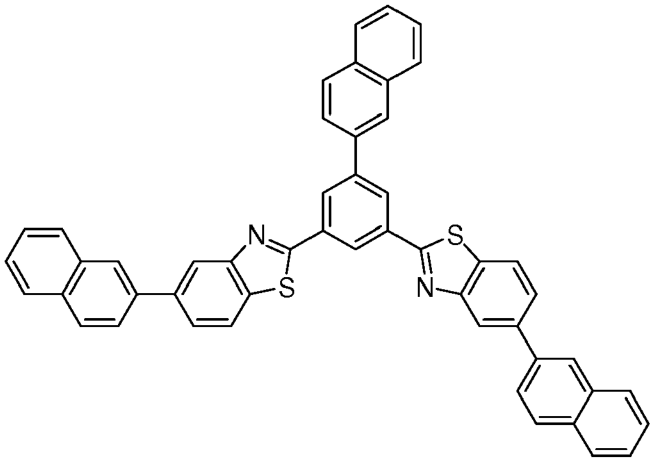

A-48 A-49

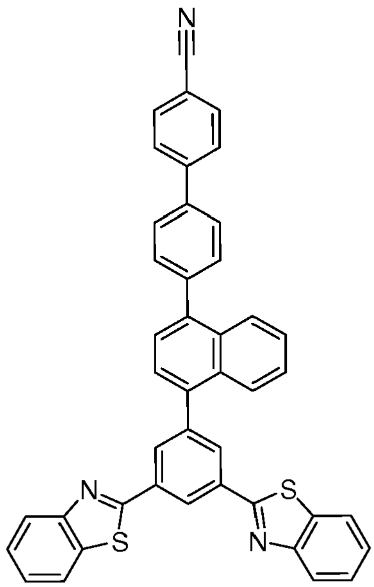

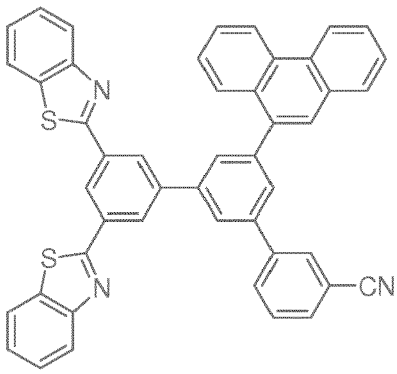

A-50 A-51

Electrical n-dopant

Under electrical n-dopant, it is understood a compound which, if embedded into an electron transport matrix, improves, in comparison with the neat matrix under the same physical condictions, the electron properties of the formed organic material, particularly in terms of electron injection and/or electron conductivity.

In the context of the present invention "embedded into an electron transport matrix" means homogenously mixed with the electron transport matrix.

The electrical n-dopant may be selected from elemental metals, metal salts, metal complexes and organic radicals.

In one embodiment, the electrical n-dopant is selected from alkali metal salts and alkali metal complexes; preferably from lithium salts and lithium organic complexes; more preferably from lithium halides and lithium organic chelates; even more preferably from lithium fluoride, a lithium quinolinolate, lithium borate, lithium phenolate, lithium pyridinolate or from a lithium complex with a Schiff base ligand; most preferably,

- the lithium quinolinolate complex has the formula II, III or IV:

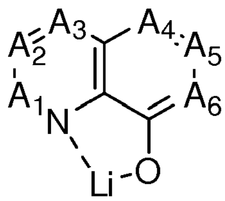 (II), 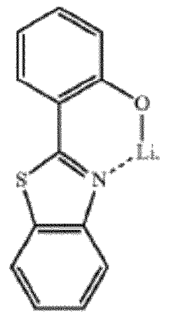 (III), 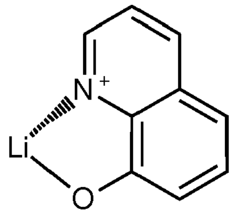 (IV)

wherein
A1 to A6 are same or independently selected from CH, CR, N, O;
R is same or independently selected from hydrogen, halogen, alkyl or aryl or heteroaryl with 1 to 20 carbon atoms; and more preferred A1 to A6 are CH,

- the borate based organic ligand is a tetra(1H-pyrazol-1-yl)borate,
- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
- the pyridinolate is a 2-(diphenylphosphoryl)pyridin-3-olate,
- the lithium Schiff base has the structure 100, 101, 102 or 103:

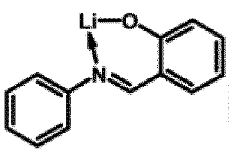 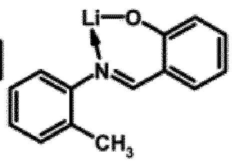 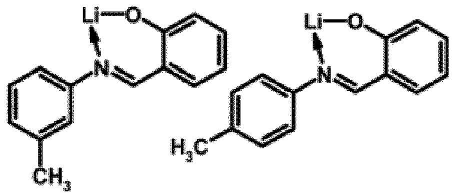

100 101 102 103 .

In another embodiment, the electrical n-dopant is a redox n-dopant.

Redox n-dopant

Under redox n-dopant, it is understood a compound which, if embedded into an electron transport matrix, increases concentration of free electrons in comparison with the neat matrix under the same physical condictions.

The redox n-dopant may not emit light under the operation condition of an electroluminescent device, for example an OLED. In one embodiment, the redox n-dopant is selected from an elemental metal, an electrically neutral metal complex and/or an electrically neutral organic radical.

The most practical benchmark for the strength of an n-dopant is the value of its redox potential. There is no particular limitation in terms how negative the value of the redox potential can be.

As reduction potentials of usual electron transport matrices used in organic semiconductors are, if measured by cyclic voltammetry against ferrocene/ferrocenium reference redox couple, roughly in the range from about - 0.8 V to about - 3.1 V; the practically applicable range of redox potentials for n-dopants which can effectively n-dope such matrices is in a slightly broader range, from about - 0.5 to about - 3.3 V.

The measurement of redox potentials is practically performed for a corresponding redox couple consisting of the reduced and of the oxidized form of the same compound. In case that the redox n-dopant is an electrically neutral metal complex and/or an electrically neutral organic radical, the measurement of its redox potential is actually performed for the redox couple formed by

(i) the electrically neutral metal complex and its cation radical formed by an abstraction of one electron from the electrically neutral metal complex, or
(ii) the electrically neutral organic radical and its cation formed by an abstraction of one electron from the electrically neutral organic radical.

Preferably, the redox potential of the electrically neutral metal complex and/or of the electrically neutral organic radical may have a value which is more negative than - 0.5 V, preferably more negative than - 1.2 V, more preferably

more negative than - 1.7 V, even more preferably more negative than - 2.1 V, most preferably more negative than - 2.5 V, if measured by cyclic voltammetry against ferrocene/ferrocenium reference redox couple for a corresponding redox couple consisting of

(i) the electrically neutral metal complex and its cation radical formed by an abstraction of one electron from the electrically neutral metal complex, or
(ii) the electrically neutral organic radical and its cation formed by an abstraction of one electron from the electrically neutral organic radical.

In a preferred embodiment, the redox potential of the n-dopant is between the value which is about 0.5 V more positive and the value which is about 0.5 V more negative than the value of the reduction potential of the chosen electron transport matrix.
Electrically neutral metal complexes suitable as redox n-dopants may be e.g. strongly reductive compelxes of some transition metals in low oxidation state. Particularly strong redox n-dopants may be selected for example from Cr(II), Mo(II) and/or W(II) guanidinate complexes such as $W_2(hpp)_4$, as described in more detail in WO2005/086251.
Electrically neutral organic radicals suitable as redox n-dopants may be e.g. organic radicals created by supply of additional energy from their stable dimers, oligomers or polymers, as described in more detail in EP 1 837 926 B1, WO2007/107306, or WO2007/107356.Under an elemental metal, it is understood a metal in a state of a neat metal, of a metal alloy, or in a state of free atoms or metal clusters. It is understood that metals deposited by vacuum thermal evaporation from a metallic phase, e.g. from a neat bulk metal, vaporize in their elemental form.
It is further understood that if the vaporized elemental metal is deposited together with a covalent matrix, the metal atoms and/or clusters are embedded in the covalent matrix. In other words, it is understood that any metal doped covalent material prepared by vacuum thermal evaporation contains the metal at least partially in its elemental form.
For the use in consumer electronics, only metals containing stable nuclides or nuclides having very long halftime of radioactive decay might be applicable. As an acceptable level of nuclear stability, the nuclear stability of natural potassium can be taken.
In one embodiment, the n-dopant may be selected from electropositive metals selected from alkali metals, alkaline earth metals, rare earth metals and metals of the first transition period Ti, V, Cr and Mn. Preferably, the n-dopant may be selected from_Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.
The redox dopant may be essentially non-emissive.
According to another aspect of the invention, it is provided an electronic device comprising a first electrode, a second electrode, and arranged between the first and second electrode, a layer comprising the organic material according to invention. The layer of the organic material according to invention may serve as a electron transport layer and/or a hole bocking layer. In one embodiment, the electronic device is an electroluminescent device. Preferably, the electroluminescent device is an organic light emitting diode.
According to another aspect of the invention, it is provided an electronic device comprising at least one electroluminescent device according to any embodiment described throughout this application, preferably, the electronic device comprises the organic light emitting diode in one of embodiments described throughout this application. More preferably, the electronic device is a display device.

**Description of the Drawings**

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.
Additional details, characteristics and advantages of the object of the invention are disclosed in the subclaims and the following description of the respective figures --which in an exemplary fashion--show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

FIG. 1 is a cross-sectional view showing an organic light emitting diode according to an embodiment of the invention.
FIGS. 2 and 3 are cross-sectional views specifically showing a part of an organic layer of an organic light emitting diode according to an embodiment of the invention.

Hereinafter, the figures are illustrated in more detail with reference to examples. However, the present disclosure is not limited to the following figures.

FIGS. 1 to 3 are schematic cross-sectional views of organic light emitting diodes 100, 300, and 400 according to an embodiment of the present invention. Hereinafter, referring to FIG 1, a structure of an organic light emitting diode according to an embodiment of the present invention and a method of manufacturing the same are as follows. The organic light emitting diode 100 has a structure where an anode 110, a stack of organic layers 105 including an optional hole transport region; an emission layer 130; and a cathode 150 that are sequentially stacked.

A substrate may be disposed on the anode 110 or under the cathode 150. The substrate may be selected from usual substrate used in a general organic light emitting diode and may be a glass substrate or a transparent plastic substrate.

The anode 110 may be formed by depositing or sputtering an anode material on a substrate. The anode material may be selected from materials having a high work function that makes hole injection easy. The anode 110 may be a reflective electrode, a transflective electrode, or a transmissive electrode. The anode material may use indium tin oxide (ITO), indium zinc oxide (IZO), tin oxide ($SnO_2$), zinc oxide (ZnO), and the like. Or, it may be a metal such as silver (Ag), or gold (Au), or an alloy thereof.

The anode 110 may have a monolayer or a multi-layer structure of two or more layers.

The organic light emitting diodes 100, 300, and 400 according to an embodiment of the present invention may include a hole transport region; an emission layer 130; and a first electron transport layer 31 comprising a compound according to formula I.

Referring to FIG. 2, the hole transport region of the stack of organic layers 105 may include at least two layered hole transport layers, and in this case, the hole transport layer contacting the emission layer (130) is defined as a second hole transport layer 135 and a the hole transport layer contacting the anode (110) is defined as a first hole transport layer 34. The stack of organic layers 105 further includes two further layers, namely a hole blocking layer 33 and a electron transport layer 31. The hole transport region of the stack 105 may further include at least one of a hole injection layer, a hole transport layer, an electron blocking layer, and a buffer layer.

The hole transport region of the stack 105 may include only hole injection layer or only hole transport layer. Or, the hole transport region may have a structure where a hole injection layer 36/hole transport layer 34 or hole injection layer 36/hole transport layer 34/electron blocking layer (135) is sequentially stacked from the anode 110.

For example, the hole injection layer 36 and the electron injection layer 37 can be additionally included, so that an OLED may comprise an anode 110/hole injection layer 36/first hole transport layer 34/electron blocking layer 135/emission layer 130/hole blocking layer 33/ electron transport layer 31/electron injection layer 37/cathode 150, which are sequentially stacked.

According to another aspect of the invention, the organic electroluminescent device (400) comprises an anode (110), a hole injection layer (36), a first hole transport layer (34), optional an electron blocking layer (135), an emission layer (130), hole blocking layer (33), electron transport layer (31), an optional electron injection layer (37), a cathode (150) wherein the layers are arranged in that order.

The hole injection layer 36 may improve interface properties between ITO as an anode and an organic material used for the hole transport layer 34, and is applied on a non-planarized ITO and thus planarizes the surface of the ITO. For example, the hole injection layer 36 may include a material having a median value of the energy level of its highest occupied molecular orbital (HOMO) between the work function of ITO and the energy level of the HOMO of the hole transport layer 34, in order to adjust a difference between the work function of ITO as an anode and the energy level of the HOMO of the first hole transport layer 34.

When the hole transport region includes a hole injection layer 36, the hole injection layer may be formed on the anode 110 by any of a variety of methods, for example, vacuum deposition, spin coating, casting, Langmuir-Blodgett (LB) method, or the like.

When hole injection layer is formed using vacuum deposition, vacuum deposition conditions may vary depending on the material that is used to form the hole injection layer, and the desired structure and thermal properties of the hole injection layer to be formed and for example, vacuum deposition may be performed at a temperature of about 100 °C to about 500 °C, a pressure of about $10^{-6}$ Pa to about $10^{-1}$ Pa, and a deposition rate of about 0.1 to about 10 nm/sec, but the deposition conditions are not limited thereto.

When the hole injection layer is formed using spin coating, the coating conditions may vary depending on the material that is used to form the hole injection layer, and the desired structure and thermal properties of the hole injection layer to be formed. For example, the coating rate may be in the range of about 2000 rpm to about 5000 rpm, and a temperature at which heat treatment is performed to remove a solvent after coating may be in a range of about 80 °C to about 200 °C, but the coating conditions are not limited thereto.

Conditions for forming the hole transport layer and the electron blocking layer may be defined based on the above-described formation conditions for the hole injection layer.

A thickness of the hole transport part of the charge transport region may be from about 10 nm to about 1000

nm, for example, about 10 nm to about 100 nm. When the hole transport transport part of the charge transport region includes the hole injection layer and the hole transport layer, a thickness of the hole injection layer may be from about 10 nm to about 1000 nm, for example about 10 nm to about 100 nm and a thickness of the hole transport layer may be from about 5 nm to about 200 nm, for example about 10 nm to about 150 nm. When the thicknesses of the hole transport part of the charge transport region, the HIL, and the HTL are within these ranges, satisfactory hole transport characteristics may be obtained without a substantial increase in driving voltage.

Hole transport matrix materials used in the hole transport region are not particularly limited. Preferred are covalent compounds comprising a conjugated system of at least 6 delocalized electrons. The term "covalent compound" is in more detail explained below, in the paragraph regarding the second electron transport matrix. Typical examples of hole transport matrix materials which are widely used in hole transport layers are polycyclic aromatic hydrocarbons, triaryl amine compounds and heterocyclic aromatic compounds. Suitable ranges of frontier orbital energy levels of hole transport matrices useful in various layer of the hole transport region are well-known. In terms of the redox potential of the redox couple HTL matrix/ cation radical of the HTL matrix, the preferred values (if measured for example by cyclic voltammetry against ferrocene/ferrocenium redox couple as reference) may be in the range 0.0 - 1.0 V, more preferably in the range 0.2 - 0.7 V, even more preferably in the range 0.3 - 0.5 V.

The hole transport region of the stack of organic layers may further include a charge-generating material to improve conductivity, in addition to the materials as described above. The charge-generating material may be homogeneously or non-homogeneously dispersed in the hole transport region.

The charge-generating material may be, for example, a p-dopant. The p-dopant may be one of a quinone derivative, a metal oxide, and a cyano group-containing compound, but is not limited thereto. Non-limiting examples of the p-dopant are quinone derivatives such as tetracyanoquinonedimethane (TCNQ), 2,3,5,6-tetrafluoro-tetracyano-1,4-benzoquinonedimethane (F4-TCNQ), and the like; metal oxides such as tungsten oxide, molybdenum oxide, and the like; and cyano-containing compounds such as compound HT-D1 below.

Compound HT-D1

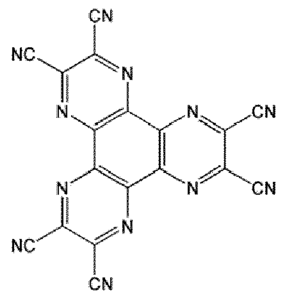

F4-TCNQ

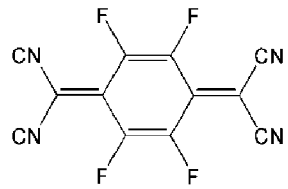

The hole transport part of the charge transport region may further include a buffer layer.

The buffer layer may compensate for an optical resonance distance of light according to a wavelength of the light emitted from the EML, and thus may increase efficiency.

The emission layer (EML) may be formed on the hole transport region by using vacuum deposition, spin coating, casting, LB method, or the like. When the emission layer is formed using vacuum deposition or spin coating, the conditions for deposition and coating may be similar to those for the formation of the hole injection layer, though the conditions for the deposition and coating may vary depending on the material that is used to form the emission layer. The emission layer may include an emitter host (EML host) and an emitter dopant (further only emitter).

The emitter may be a red, green, or blue emitter.

In one embodiment, the emitter host is an anthracene matrix compound represented by formula 400 below:

Formula 400

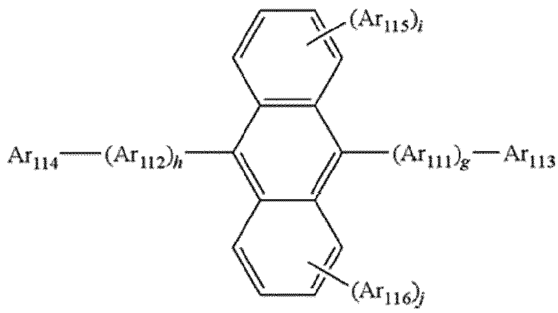

In formula 400, Arm and $Ar_{112}$ may be each independently a substituted or unsubstituted $C_6$-$C_{60}$ arylene group; $Ar_{113}$ to $Ar_{116}$ may be each independently a substituted or unsubstituted $C_1$-$C_{10}$ alkyl group or a substituted or unsubstituted $C_6$-$C_{60}$ aryl group; and g, h, i, and j may be each independently an integer from 0 to 4. In some embodiments, $Ar_{111}$ and $Ar_{112}$ in formula 400 may be each independently one of a phenylene group, a naphthylene group, a phenanthrenylene group, or a pyrenylene group; or a phenylene group, a naphthylene group, a phenanthrenylene group, a fluorenyl group, or a pyrenylene group, each substituted with at least one of a phenyl group, a naphthyl group, or an anthryl group.

In formula 400, g, h, i, and j may be each independently an integer of 0, 1, or 2. In formula 400, $Ar_{113}$ to $Ar_{116}$ may be each independently one of

- a $C_1$-$C_{10}$ alkyl group substituted with at least one of a phenyl group, a naphthyl group, or an anthryl group;
- a phenyl group, a naphthyl group, an anthryl group, a pyrenyl group, a phenanthrenyl group, or a fluorenyl group;
- a phenyl group, a naphthyl group, an anthryl group, a pyrenyl group, a phenanthrenyl group, or
- a fluorenyl group, each substituted with at least one of a deuterium atom, a halogen atom, a hydroxyl group, a cyano group, a nitro group, an amino group, an amidino group, a hydrazine group, a hydrazone group, a carboxyl group or a salt thereof,
- a sulfonic acid group or a salt thereof,
- a phosphoric acid group or a salt thereof,
- a $C_1$-$C_{60}$ alkyl group, a $C_2$-$C_{60}$ alkenyl group, a $C_2$-$C_{60}$ alkynyl group, a $C_1$-$C_{60}$ alkoxy group, a phenyl group, a naphthyl group, an anthryl group, a pyrenyl group, a phenanthrenyl group, or
- a fluorenyl group; or

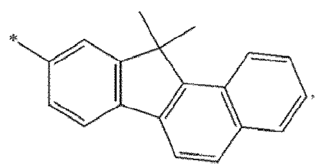

or formulas (Y2) or (Y3):

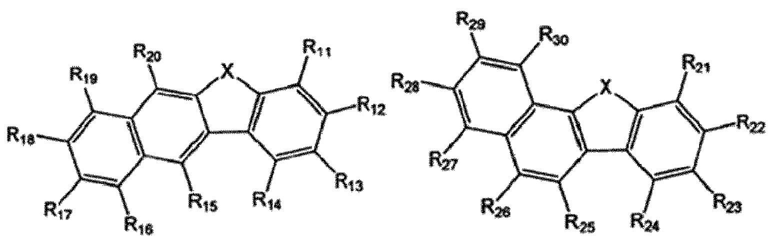

(Y2), (Y3).

Wherein in the formulas (Y2) and (Y3), X is selected form an oxygen atom and a sulfur atom, but embodiments of the invention are not limited thereto.

In the formula (Y2), any one of $R_{11}$ to $R_{14}$ is used for bonding to $Ar_{111}$. $R_{11}$ to $R_{14}$ that are not used for bonding

to Arm and $R_{15}$ to $R_{20}$ are the same as $R_1$ to $R_8$.

In the formula (Y3), any one of $R_{21}$ to $R_{24}$ is used for bonding to Arm. $R_{21}$ to $R_{24}$ that are not used for bonding to Arm and $R_{25}$ to $R_{30}$ are the same as $R_1$ to $R_8$.

Preferably, the EML host comprises between one and three heteroatoms selected from the group consisting of N, O or S. More preferred the EML host comprises one heteroatom selected from S or O.

According to a further aspect of the invention, the emitter host respectively has a reduction potential which, if measured under the same conditions by cyclic voltammetry against $Fc/Fc^+$ in tetrahydrofuran, has a value more negative than the respective value obtained for 7-([1,1'-biphenyl]-4-yl)dibenzo[c,h]acridine, preferably more negative than the respective value for 9,9',10,10'-tetraphenyl-2,2'-bianthracene, more preferably more negative than the respective value for 2,9-di([1,1'-biphenyl]-4-yl)-4,7-diphenyl-1,10-phenanthroline, even more preferably more negative than the respective value for 2,4,7,9-tetraphenyl-1,10-phenanthroline, even more preferably more negative than the respective value for 9,10-di(naphthalen-2-yl)-2-phenylanthracene, even more preferably more negative than the respective value for 2,9-bis(2-methoxyphenyl)-4,7-diphenyl-1,10-phenanthroline, most preferably more negative than the respective value for 9,9'-spirobi[fluorene]-2,7-diylbis(diphenylphosphine oxide).

The emitter is mixed in a small amount to cause light emission, and may be generally a material such as a metal complex that emits light by multiple excitation into a triplet or more. The emitter may be, for example an inorganic, organic, or organometallic compound, and one or more kinds thereof may be used.

The emitter may be a fluorescent emitter, for example ter-fluorene, the structures are shown below. 4.4'-bis(4-diphenyl amiostyryl)biphenyl (DPAVBi), 2,5,8,11-tetra-tert-butyl perylene (TBPe), and Compound 4 below are examples of fluorescent blue emitters.

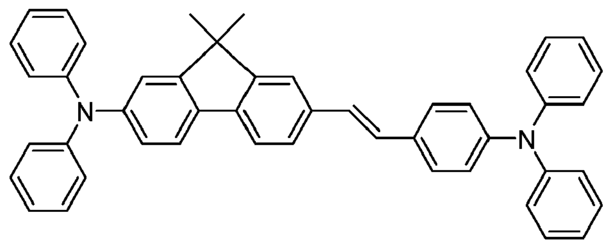

**Compound 4**

According to another aspect, the organic semiconductor layer comprising a compound of formula I is arranged between a fluorescent blue emission layer and the cathode electrode.

The emitter may be a phosphorescent emitter, and examples of the phosphorescent emitters may be organometallic compounds including Ir, Pt, Os, Ti, Zr, Hf, Eu, Tb, Tm, Fe, Co, Ni, Ru, Rh, Pd, or a combination thereof. The phosphorescent emitter may be, for example a compound represented by formula Z, but is not limited thereto:

$$L_2MX \text{ (Z).}$$

In formula Z, M is a metal, and L and X are the same or different, and are a ligand to form a complex compound with M.

The M may be, for example Ir, Pt, Os, Ti, Zr, Hf, Eu, Tb, Tm, Fe, Co, Ni, Ru, Rh, Pd or, in a polynuclear complex,a combination thereof, and the L and X may be, for example, a bidendate ligand.

A thickness of the emission layer may be about 10 nm to about 100 nm, for example about 20 nm to about 60 nm. When the thickness of the emission layer is within these ranges, the emission layer may have improved emission characteristics without a substantial increase in a driving voltage.

Next, the electron transport region of the stack of organic layers 105 is disposed on the emission layer.

The electron transport region of the stack of organic layers includes at least an electron transport layer. The electron transport region of the stack of organic layers may further include an electron injection layer and/or a hole blocking layer. At least an electron transport layer comprises the n-doped organic material according to one of its various embodiments.

For example, the electron transport region of the stack of organic layers may have a structure of an electron transport layer/hole blocking layer/electron injection layer but is not limited thereto. For example, an organic light emitting diode according to an embodiment of the present invention includes at least two electron transport layers in the electron transport region of the stack of organic layers 105, and in this case, the layer contacting the emission layer is a hole blocking layer 33.

The electron transport layer may include two or more different electron transport matrix compounds.

Second electron transport matrix compound

Various embodiments of the electron transport region in the device according to invention, e.g. devices comprising hole blocking layers, electron injecting layers, may comprise a second electron transport matrix compound.

Second electron transport matrix is not particularly limited. Similarly as other materials which are in the inventive device comprised outside the emitting layer, the second electron transport matrix may not emit light.

According to one embodiment, the second electron transport matrix can be an organic compound, an organometallic compound, or a metal complex.

According to one embodiment, the second electron transport matrix may be a covalent compound comprising a conjugated system of at least 6 delocalized electrons. Under a covalent material in a broadest possible sense, it might be understood a material, wherein at least 50 % of all chemical bonds are covalent bonds, wherein coordination bonds are also considered as covalent bonds. In the present application, the term encompasses in the broadest sense all usual electron transport matrices which are predominantly selected from organic compounds but also e.g. from compounds comprising structural moieties which do not comprise carbon, for example substituted 2,4,6-tribora-1,3,5 triazines, or from metal complexes, for example aluminium tris(8-hydroxyquinolinolate).

The molecular covalent materials can comprise low molecular weight compounds which may be, preferably, stable enough to be processable by vacuum thermal evaporation (VTE). Alternatively, covalent materials can comprise polymeric covalent compounds, preferably, compounds soluble in a solvent and thus processable in form of a solution. It is to be understood that a polymeric substantially covalent material may be crosslinked to form an infinite irregular network, however, it is supposed that such crosslinked polymeric substantially covalent matrix compound still comprises both skeletal as well as peripheral atoms. Skeletal atoms of the covalent compound are covalently bound to at least two neighbour atoms. Other atoms of the covalent compound are peripheral atoms which are covalently bound with a single neighbour atom. Inorganic infinite crystals or fully crosslinked networks having partly covalent bonding but substantially lacking peripheral atoms, like silicon, germanium, gallium arsenide, indium phosphide, zinc sulfide, silicate glass etc. are not considered as covalent matrices in the sense of present application, because such fully crosslinked covalent materials comprise peripheral atoms only on the surface of the phase formed by such material. A compound comprising cations and anions is still considered as covalent, if at least the cation or at least the anion comprises at least ten covalently bound atoms.

Preferred examples of covalent second electron transport matrix compounds are organic compounds, consisting predominantly from covalently bound C, H, O, N, S, which may optionally comprise also covalently bound B, P, As, Se. In one embodiment, the second electron transport matrix compound lacks metal atoms and majority of its skeletal atoms is selected from C, O, S, N.

In another embodiment, the second electron transport matrix compound comprises a conjugated system of at least six, more preferably at least ten, even more preferably at least fourteen delocalized electrons.

Examples of conjugated systems of delocalized electrons are systems of alternating pi- and sigma bonds. Optionally, one or more two-atom structural units having the pi-bond between its atoms can be replaced by an atom bearing at least one lone electron pair, typically by a divalent atom selected from O, S, Se, Te or by a trivalent atom selected from N, P, As, Sb, Bi. Preferably, the conjugated system of delocalized electrons comprises at least one aromatic or heteroaromatic ring adhering to the Hückel rule. Also preferably, the second electron transport matrix compound may comprise at least two aromatic or heteroaromatic rings which are either linked by a covalent bond or condensed.

In one of specific embodiments, the second electron transport matrix compound comprises a ring consisting of covalently bound atoms and at least one atom in the ring is phosphorus.

In a more preferred embodiment, the phosphorus-containing ring consisting of covalently bound atoms is a phosphepine ring.

In another preferred embodiment, the covalent matrix compound comprises a phosphine oxide group. Also preferably, the substantially covalent matrix compound comprises a heterocyclic ring comprising at least one nitrogen atom. Examples of nitrogen containing heterocyclic compounds which are particularly advantageous as second electron transport matrix compound for the inventive device are matrices comprising, alone or in combination, pyridine structural moieties, diazine structural moieties, triazine structural moieties, quinoline structural moieties, benzoquinoline structural moieties, quinazoline structural moieties, acridine structural moieties, benzacridine structural moieties, dibenzacridine structural moieties, diazole structural moieties and benzodiazole structural moieties.

The second matrix compound may have a molecular weight (Mw) of $\geq$ 400 to $\leq$ 850 g / mol, preferably $\geq$ 450 to $\leq$ 830 g / mol. If the molecular weight is selected in this range, particularly reproducible evaporation and deposition can be achieved in vacuum at temperatures where good long-term stability is observed.

Preferably, the second matrix compound may be essentially non-emissive.

According to another aspect, the reduction potential of the second electron transport compound may be selected more negative than -2.2 V and less negative than -2.35 V against $Fc/Fc^+$ in tetrahydrofuran, preferably more negative than -2.25 V and less negative than -2.3 V.

According to one embodiment, the first and the second matrix compound may be selected different, and

- the second electron transport layer consist of a second matrix compound; and
- the first electron transport layer consist of the organic material of formula (I), and an electrical n-dopant, preferably an alkali metal salt or an alkali metal organic complex.

Preferably, the first and second electron transport layer may be essentially non-emissive.
According to one embodiment the hole blocking layer may comprise the organic material.
According to another embodiment, the second electron transport layer can be in direct contact with the emission layer.
According to another embodiment, the electron transport layer can be in direct contact with a hole blocking layer.
According to another embodiment, the second electron transport layer can be contacting sandwiched between the emission layer and the first electron transport layer.
According to another embodiment, the first electron transport layer can be in direct contact with the electron injection layer.
According to another embodiment, the first electron transport layer can be contacting sandwiched between the second electron transport layer and the electron injection layer.
According to another embodiment, the first electron transport layer can be in direct contact with the cathode electrode.
According to another embodiment, the first electron transport layer can be contacting sandwiched between the second electron transport layer and the cathode layer.
According to another embodiment, the second electron transport layer can be contacting sandwiched between the emission layer and the first electron transport layer, and the first electron transport layer can be contacting sandwiched between the second electron transport layer and the electron injection layer or sandwiched between the second electron transport layer and the hole blocking layer
The formation conditions of the first electron transport layer 31, hole blocking layer 33, and electron injection layer 37 of the electron transport region of the stack of organic layers refer to the formation conditions of the hole injection layer.
The thickness of the first electron transport layer may be from about 2 nm to about 100 nm, for example about 3 nm to about 30 nm. When the thickness of the first electron transport layer is within these ranges, the first electron transport layer may have improved electron transport auxiliary ability without a substantial increase in driving voltage.
A thickness of the second electron transport layer may be about 10 nm to about 100 nm, for example about 15 nm to about 50 nm. When the thickness of the electron transport layer is within these ranges, the electron transport layer may have satisfactory electron transporting ability without a substantial increase in driving voltage.
According to another aspect of the invention, the organic electroluminescent device further comprises an electron injection layer between the second electron transport layer and the cathode.
The electron injection layer (EIL) 37 may facilitate injection of electrons from the cathode 150.
According to another aspect of the invention, the electron injection layer 37 comprises:

(i) an electropositive metal selected from alkali metals, alkaline earth metals and rare earth metals in substantially elemental form, preferably selected from Li, Na, K, Rb, Cs, Mg, Ca, Sr, Ba, Eu and Yb, more preferably from Li, Na, Mg, Ca, Sr and Yb, even more preferably from Li and Yb, most preferably Yb; and/or
(ii) an alkali metal complex and/or alkali metal salt, preferably the Li complex and/or salt, more preferably a Li quinolinolate, even more preferably a lithium 8-hydroxyquinolinolate, most preferably the alkali metal salt and/or complex of the second electron transport layer is identicial with the alkali metal salt and/or complex of the injection layer.

The electron injection layer may include at least one selected from LiF, NaCl, CsF, $Li_2O$, and BaO.
A thickness of the EIL may be from about 0.1 nm to about 10 nm, or about 0.3 nm to about 9 nm. When the thickness of the electron injection layer is within these ranges, the electron injection layer may have satisfactory electron injection ability without a substantial increase in driving voltage.
A material for the cathode 150 may be a metal, an alloy, or an electrically conductive compound that have a low work function, or a combination thereof. Specific examples of the material for the cathode 150 may be lithium (Li), magnesium (Mg), aluminum (Al), aluminum-lithium (Al-Li), calcium (Ca), magnesium-indium (Mg-In), magnesium-silver (Mg-Ag), etc. In order to manufacture a top-emission light-emitting device having a reflective anode 110 deposited on a substrate, the cathode 150 may be formed as a transmissive electrode from, for example, indium tin oxide (ITO) or indium zinc oxide (IZO).
In one embodiment, the organic electronic device according to the invention comprising an organic semiconducting layer comprising a compound according to Formula (I) can further comprise a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection layer, hole transporting and/or a hole generation layer the later one having the function of generating holes in a charge-generation layer or a p-n-junction.

In one embodiment, the radialene compound may have formula (XX) and/or the quinodimethane compound may have formula (XXIa) or (XXIb):

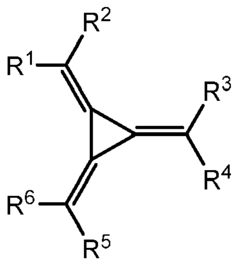
(XX)

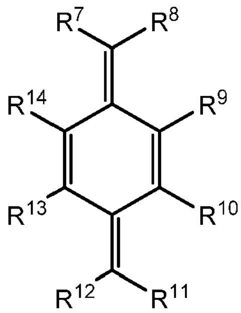
(XXIa)

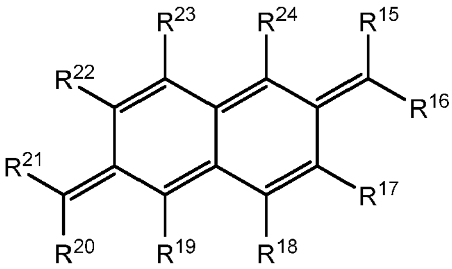
(XXIb),

wherein $R^1$, $R^2$, $R^3$, $R^4$, $R^5$, $R^6$, $R^7$, $R^8$, $R^{11}$, $R^{12}$, $R^{15}$, $R^{16}$, $R^{20}$, $R^{21}$ are independently selected from above mentioned electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and above mentioned electron withdrawing groups.

Hereinafter, the embodiments are illustrated in more detail with reference to examples. However, the present disclosure is not limited to the following examples.

**Detailed description**

The invention is furthermore illustrated by the following examples which are illustrative only and non-binding.

**Synthesis of compounds of Formula (1)**

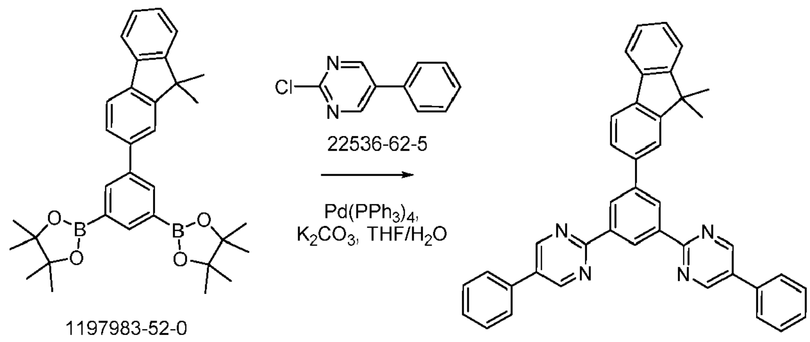


**2,2'-(5-(9,9-dimethyl-9H-fluoren-2-yl)-1,3-phenylene)bis(5-phenylpyrimidine)**

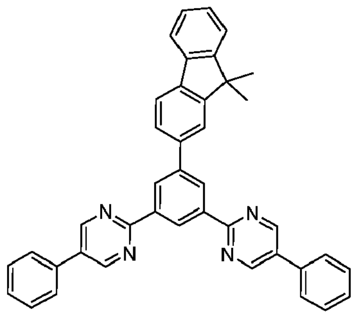

A flask was flushed with nitrogen and charged with 2-chloro-5-phenylpyrimidine (7.7 g, 40.2 mmol), 2,2'-(5-(9,9-dimethyl-9H-fluoren-2-yl)-1,3-phenylene)bis(4,4,5,5-tetramethyl-1,3,2-dioxaborolane) (10 g, 19.2 mmol), $Pd(PPh_3)_4$ (0.44 g, 0.38 mmol), and $K_2CO_3$ (5.3 g, 38 mmol). A mixture of deaerated THF/water (3:1, 80 mL) was added and the reaction mixture was stirred at reflux temperature under a nitrogen atmosphere for 46 h. After cooling down to ambient temperature, the resulting precipitate was isolated by suction filtration and washed with n-hexane. The obtained white solid was dissolved in dichloromethane and washed with water three times. After drying over $MgSO_4$, the organic phase was filtered over a pad of Florisil. After rinsing with additional dichloromethane, the colorless filtrate was concentrated in vacuo to a minimal volume and n-hexane was added. The resulting white precipitate was isolated by suction filtration and washed with n-hexane. Further purification was achieved by trituration with n-hexane/dichloromethane (9:1) and recrystallization from toluene to afford 8.6 g (78%) of a white solid after drying. Final purification was achieved by sublimation. HPLC: 99.8%; ESI-MS: m/z = 579 ($[M+H]^+$).

**Synthesis of inventive compound 2:**

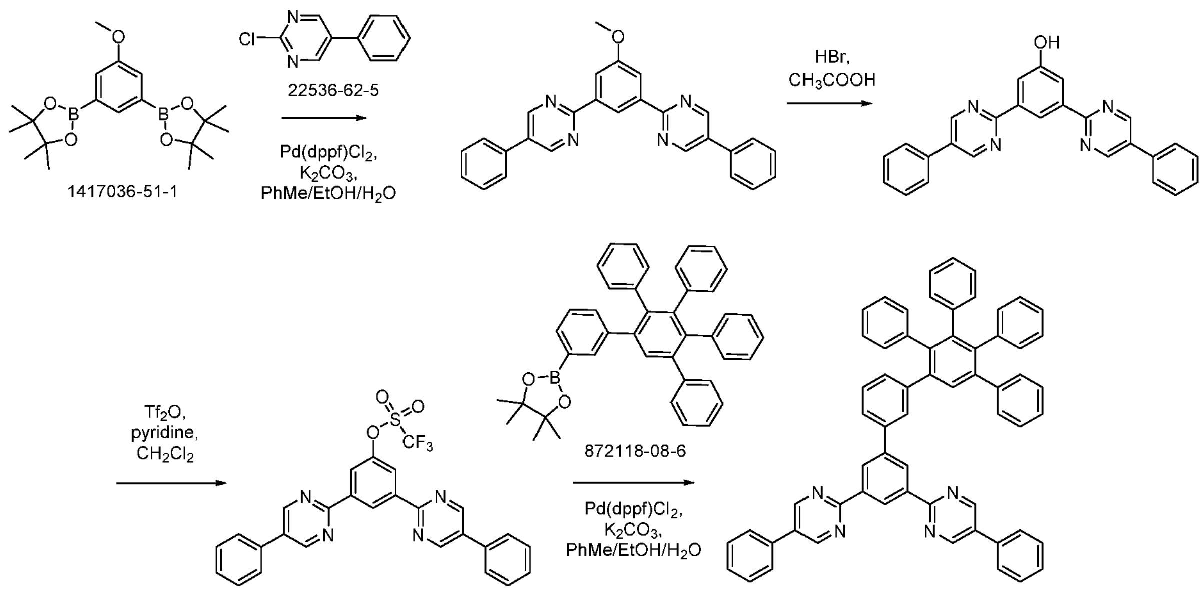


**2,2'-(5-methoxy-1,3-phenylene)bis(5-phenylpyrimidine)**

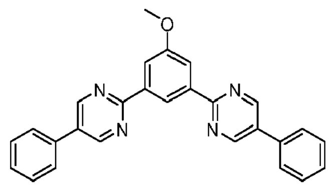

A flask was flushed with nitrogen and charged with 2-chloro-5-phenylpyrimidine (19.1 g, 100 mmol), 2,2'-(5-methoxy-1,3-phenylene)bis(4,4,5,5-tetramethyl-1,3,2-dioxaborolane) (15 g, 41.7 mmol), Pd(dppf)$Cl_2$ (0.31 g, 0.4 mmol), and $K_2CO_3$ (23 g, 166.4 mmol). A mixture of deaerated toluene/ethanol/water (3:1:1, 410 mL) was added and the reaction mixture was stirred at reflux temperature under a nitrogen atmosphere for 22 h. After cooling down to ambient temperature, all volatiles were removed in vacuo and the resulting solid was dissolved in chloroform/water. The organic phase was washed with water three times, dried over $MgSO_4$ and filtered over a pad of Florisil. After rinsing with additional chloroform, the filtrate was concentrated in vacuo to a minimal volume and n-hexane was added. The resulting off-white precipitate was isolated by suction filtration and washed with n-hexane. Further purification was achieved by precipitation from dichloromethane by addition of n-hexane to afford 12.6 g (73%) of a white solid after drying. HPLC: 99.8%; GC-MS: m/z = 416.

**3,5-bis(5-phenylpyrimidin-2-yl)phenol**

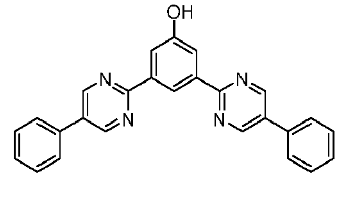

A flask was charged with 2,2'-(5-methoxy-1,3-phenylene)bis(5-phenylpyrimidine) (11.4 g, 27.4 mmol), glacial acetic acid (400 mL) and aq. HBr (69.5 mL, 48 wt% aq. solution). The resulting suspension was stirred at reflux temperature for 5 d. After cooling down to ambient temperature, the solid was isolated by suction filtration and washed with water and n-hexane. After drying in vacuo, 8.8 g (80%) of a pale yellow were obtained. HPLC: 99.6%.

**3,5-bis(5-phenylpyrimidin-2-yl)phenyl trifluoromethanesulfonate**

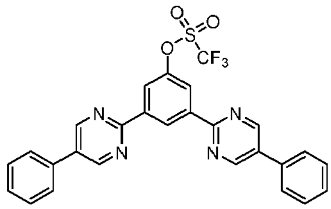

A flask was flushed with nitrogen and charged with 3,5-bis(5-phenylpyrimidin-2-yl)phenol (8.6 g, 21.4 mmol), dry dichloromethane (300 mL), and pyridine (3.45 mL, 42.7 mmol). After cooling down to 0 °C, trifluoromethanesulfonic anhydride (8.6 mL, 51.3 mmol) was added dropwise. After 30 min. at 0 °C, the reaction mixture was allowed to reach room temperature and stirring was continued for 23 h. Subsequently, aq. HCl (2 mL, 2 M) was added and the resulting solution was stirred for 10 min. After neutralization with saturated aq. $NaHCO_3$, the organic phase was washed with water five times, dried over $MgSO_4$, filtered and evaporated to dryness. The crude product was purified by trituration with n-hexane followed by silica gel filtration using dichloromethane as solvent to afford 9.7 g (85%) off a white solid after drying. HPLC: 99.2%.

**2,2'-(4',5',6'-triphenyl-[1,1':2',1":3",1"'-quaterphenyl]-3"',5"'-diyl)bis(5-phenylpyrimidine)**

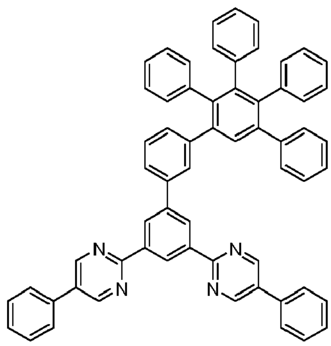

A flask was flushed with nitrogen and charged with 3,5-bis(5-phenylpyrimidin-2-yl)phenyl trifluoromethanesulfonate (9.5 g, 17.8 mmol), 4,4,5,5-tetramethyl-2-(3',4',5'-triphenyl-[1,1':2',1"-terphenyl]-3-yl)-1,3,2-dioxaborolane (12.5 g, 21.4 mmol), Pd(dppf)$Cl_2$ (0.3 g, 0.4 mmol), and $K_2CO_3$ (10.8 g, 78.1 mmol). A mixture of deaerated toluene/ethanol/water (5:1.5:1, 150 mL) was added and the reaction mixture was stirred at reflux temperature under a nitrogen atmosphere for 5 d. After cooling down to ambient temperature, the resulting precipitate was isolated by suction filtration and washed with water and n-hexane. The obtained white solid was dissolved in chloroform and washed with water three times. After drying over $MgSO_4$, the organic phase was filtered over a pad of silica. After rinsing with additional chloroform, the colorless filtrate was concentrated in vacuo to a minimal volume and n-hexane was added. The resulting white precipitate was isolated by suction filtration and washed with n-hexane to afford 2.5 g (17%) of a white solid after drying. Final purification was achieved by sublimation. HPLC: 100%; ESI-MS: m/z = 843 ($[M+H]^+$).

**Synthesis of inventive compounds 3 and 4:**

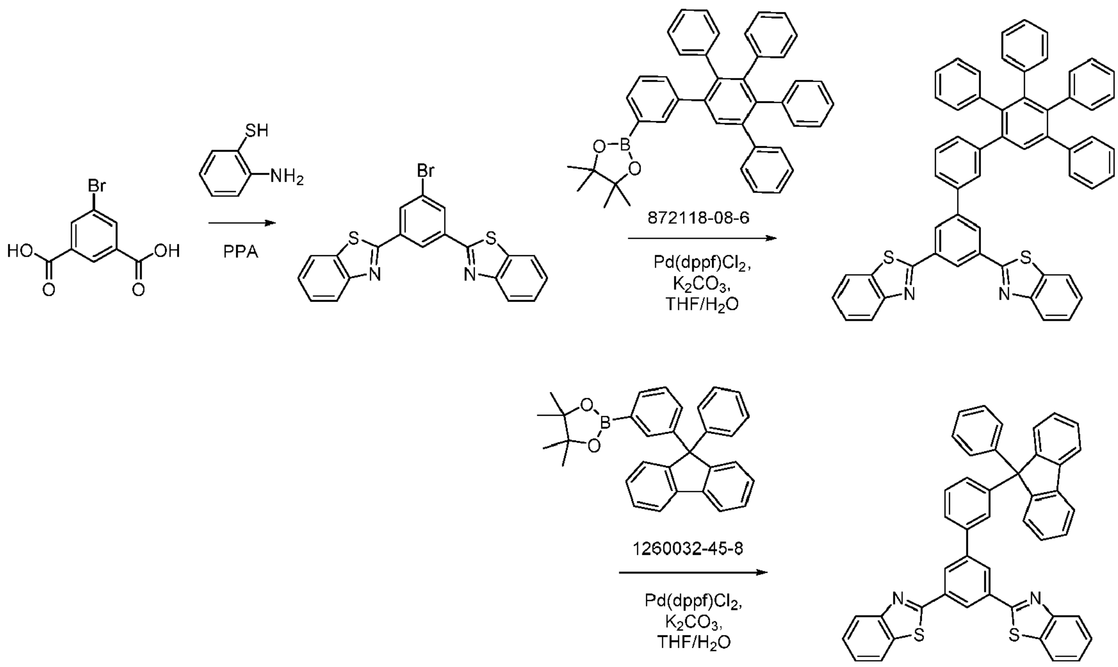


**2,2'-(5-bromo-1,3-phenylene)bis(benzo[d]thiazole)**

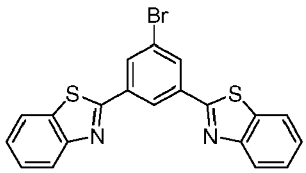

A flask equipped with a mechanical stirrer was charged with polyphosphoric acid (800 mL), 2-aminobenzenethiol (53.6 g, 428.5 mmol), and 5-bromoisophthalic acid (50 g, 204 mmol). The mixture was stirred at 230 °C overnight. The reaction mixture was allowed to cool down to 130 °C and carefully poured into an excess of ice water. The resulting suspension was stirred for 2 h. Subsequently, the precipitate was isolated by suction filtration and washed with water until the filtrate was pH neutral. The solid was triturated with methanol and dried. Subsequently, the solid was dissolved in hot chlorobenzene and filtered over a pad of silica. After rinsing with additional hot chlorobenzene, the filtrate was concentrated in vacuo and the resulting precipitate was isolated by suction filtration, washed with toluene and methanol and dried to afford 74 g (86%) of a pale yellow solid. HPLC: 99.7%; ESI-MS: m/z = 424 ($[M+H]^+$). Analog procedure: N. Chandrashekhar et al., Magn. Reson. Chem. 2008, 46, 769-774

**2,2'-(4',5',6'-triphenyl-[1,1':2',1":3",1"'-quaterphenyl]-3"',5"'-diyl)bis(benzo[d]thiazole)**

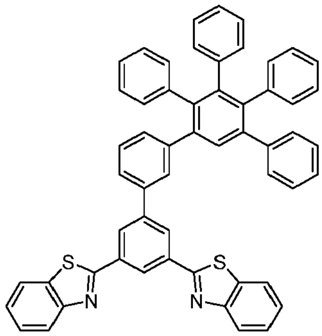

A flask was flushed with nitrogen and charged with 2,2'-(5-bromo-1,3-phenylene)bis(benzo[d]thiazole) (6.8 g, 16.1 mmol), 4,4,5,5-tetramethyl-2-(3',4',5'-triphenyl-[1,1':2',1"-terphenyl]-3-yl)-1,3,2-dioxaborolane (10.3 g, 17.7 mmol), $Pd(dppf)Cl_2$ (0.24 g, 0.33 mmol), and $K_2CO_3$ (4.4 g, 32.1 mmol). A mixture of deaerated THF/water (15.6:1, 266 mL) was added and the reaction mixture was stirred under a nitrogen atmosphere at reflux temperature for 140 h. Subsequently, all volatiles were removed in vacuo and the obtained solid was dissolved in chloroform/water. The organic phase was washed with water three times, dried over $MgSO_4$ and filtered over a pad of Florisil. After rinsing with additional chloroform, the colorless filtrate was concentrated in vacuo to a minimal volume and cyclohexane was added. After stirring for 30 min., the resulting white precipitate was isolated by suction filtration and washed with cyclohexane. Further purification was achieved by repeated recrystallization from chloroform/cyclohexane to afford 6 g (47%) of a white solid after drying. Final purification was achieved by sublimation. HPLC: 99.7%; ESI-MS: m/z = 801 ($[M+H]^+$).

**2,2'-(3'-(9-phenyl-9H-fluoren-9-yl)-[1,1'-biphenyl]-3,5-diyl)bis(benzo[d]thiazole)**

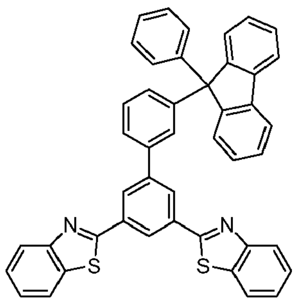

Following the procedure described above for 2,2'-(4',5',6'-triphenyl-[1,1':2',1":3",1"'-quaterphenyl]-3"',5"'-diyl)bis(benzo[d]thiazole) using 2,2'-(5-bromo-1,3-phenylene)bis(benzo[d]thiazole) (9.5 g, 22.4 mmol), 4,4,5,5-tetramethyl-2-(3-(9-phenyl-9H-fluoren-9-yl)phenyl)-1,3,2-dioxaborolane (11 g, 24.6 mmol), $Pd(dppf)Cl_2$ (0.33 g, 0.45 mmol), $K_2CO_3$ (6.2 g, 44.8 mmol), a mixture of deaerated THF/water (16:1, 372 mL), 66 h reaction time and purification by column chromatography (silica, toluene/n-hexane 5:1) afforded 9.2 g (62%) of a white solid after drying. Final purification was achieved by sublimation. HPLC: 99.7%; ESI-MS: m/z = 661 ($[M+H]^+$).

**Synthesis of inventive compound 5:**

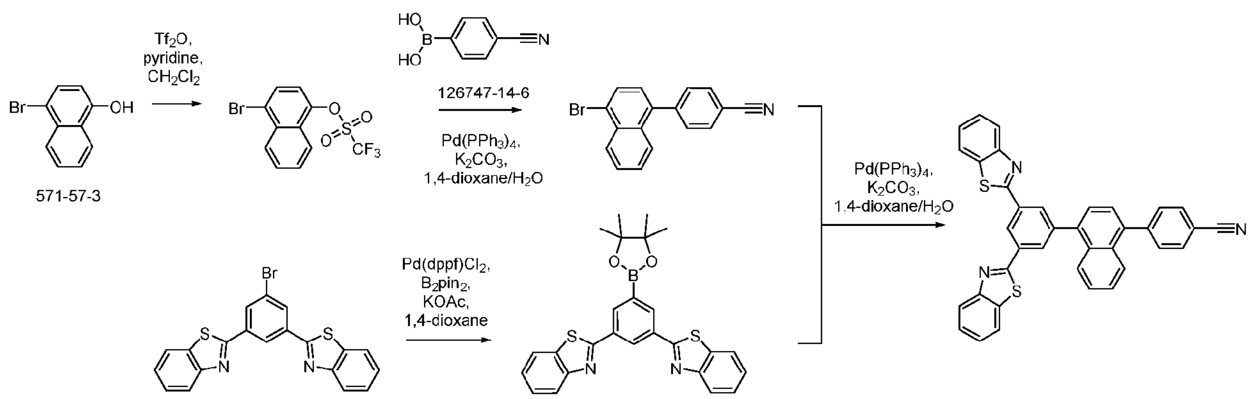


**4-bromonaphthalen-1-yl trifluoromethanesulfonate**

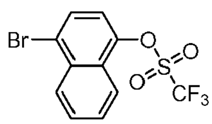

A flask was flushed with nitrogen and charged with 4-bromonaphthalen-1-ol (45 g, 201.7 mmol), pyridine (32.6 mL, 403.5 mmol) and dry dichloromethane (500 mL). After cooling down to 0 °C, trifluoromethanesulfonic anhydride (40.7 mL, 242.1 mmol) was added dropwise within 35 min while stirring. After 3 h at 0 °C, the reaction mixture was allowed to reach room temperature and stirring was continued overnight. Subsequently, water was added and the organic phase was separated. After washing with water two times, the organic phase was dried over $MgSO_4$, filtered and concentrated. After dilution with n-hexane, silica was added to absorb polar impurities and the obtained suspension was filtered. The filtrate was evaporated to dryness to afford 61 g (85%) off yellow oil. GC-FID: 99.8%; GC-MS: m/z = 355.

4-(**4-bromonaphthalen-1-yl)benzonitrile**

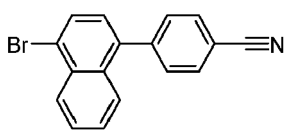

A flask was flushed with nitrogen and charged with 4-bromonaphthalen-1-yl trifluoromethanesulfonate (57.3 g, 161.4 mmol), (4-cyanophenyl)boronic acid (23.7 g, 161.4 mmol), $Pd(PPh_3)_4$ (3.7 g, 3.23 mmol), and $K_2CO_3$ (66.9 g, 484.1 mmol). A mixture of deaerated 1,4-dioxane/water (3.3:1, 1050 mL) was added and the reaction mixture was stirred at 70 °C under a nitrogen atmosphere for 9 h. After cooling down to ambient temperature, the aqueous phase was removed and the organic phase was evaporated to dryness. The obtained solid was suspended in dichloromethane and filtered. The filtrate was evaporated to dryness and the remaining solid was purified by column chromatography (silica, dichloromethane/n-hexane 2:3 to 3:2) to obtain 5.3 g (9%) of an off-white solid that was used without further purification. HPLC: 99.95%; GC-MS: m/z = 308.

**2,2'-(5-(4,4,5,5-tetramethyl-1,3,2-dioxaborolan-2-yl)-1,3-phenylene)bis(benzo[d]thiazole)**

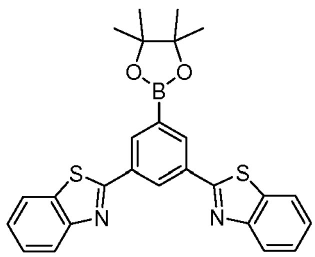

A flask was flushed with nitrogen and charged with 2,2'-(5-bromo-1,3-phenylene)bis(benzo[d]thiazole) (16.8 g, 39.7 mmol), 4,4,4',4',5,5,5',5'-octamethyl-2,2'-bi(1,3,2-dioxaborolane) (13.1 g, 51.6 mmol), Pd(dppf)$Cl_2$ (0.87 g, 1.2 mmol), potassium acetate (11.7 g, 119 mmol), and dry and deaerated 1,4-dioxane (390 mL). The resulting mixture was stirred at 80 °C under a nitrogen atmosphere for 21 h. After cooling down to ambient temperature, all volatiles were removed in vacuo and the resulting solid was dissolved in dichloromethane/water. The organic phase was washed with water three times, dried over $MgSO_4$ and filtered over a pad of Florisil. After rinsing with additional dichloromethane, the filtrate was concentrated in vacuo and h-hexane was added. The obtained solid was isolated by suction filtration, washed with n-hexane and dried to afford 15.8 g (85%) of an off-white solid. HPLC: 96%; GC-MS: m/z = 470.

**4-(4-(3,5-bis(benzo[d]thiazol-2-yl)phenyl)naphthalen-1-yl)benzonitrile**

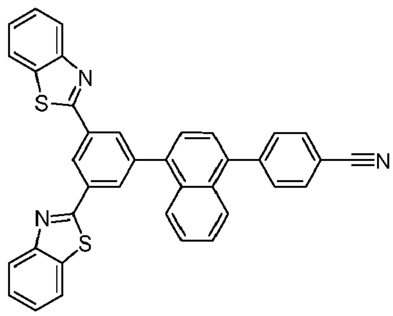

A flask was flushed with nitrogen and charged with 2,2'-(5-(4,4,5,5-tetramethyl-1,3,2-dioxaborolan-2-yl)-1,3-phenylene)bis-(benzo[d]thiazole) (8.0 g, 17 mmol), 4-(4-bromonaphthalen-1-yl)benzonitrile (5.2 g, 17 mmol), $Pd(PPh_3)_4$ (0.6 g, 0.5 mmol), and $K_2CO_3$ (7.1 g, 51 mmol). A mixture of deaerated 1,4-dioxane/water (4:1, 125 mL) was added and the reaction mixture was stirred at 90 °C under a nitrogen atmosphere for 5 h. After cooling down to ambient temperature, the resulting precipitate was isolated by suction filtration and washed with 1,4-dioxane and water until the filtrate was pH neutral. After drying, the obtained solid was dissolved in hot chloroform and filtered over a pad of silica gel. After rinsing with additional chloroform, the colorless filtrate was concentrated in vacuo and h-heptane was added. The obtained solid was isolated by suction filtration and washed with n-heptane. Further purification was achieved by recrystallization from chlorobenzene to yield 5.8 g (60%) of a white solid after drying. Final purification was achieved by sublimation. HPLC: 99.7%; ESI-MS: m/z = 572 ($[M+H]^+$).

**Synthesis of inventive compound 6:**

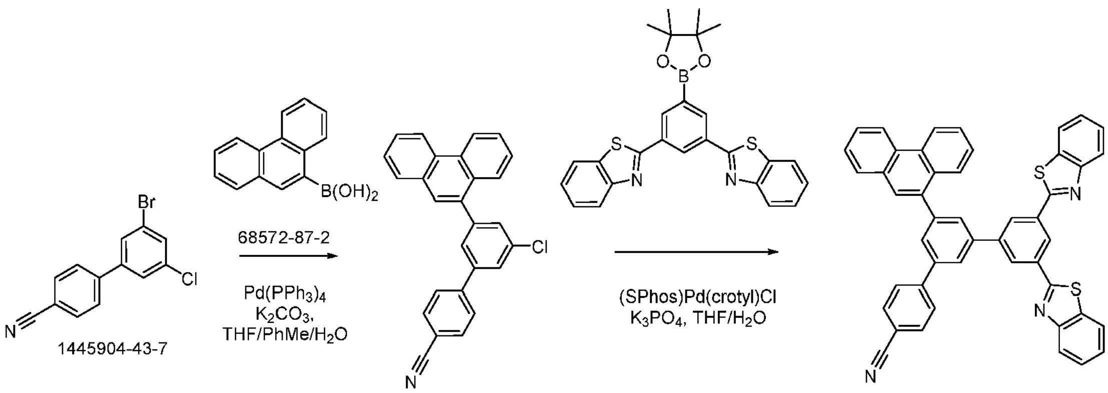


**3'-chloro-5'-(phenanthren-9-yl)-[1,1'-biphenyl]-4-carbonitrile**

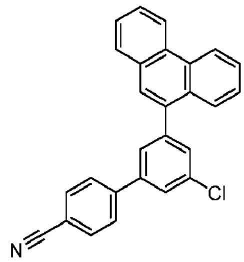

A flask was flushed with nitrogen and charged with 3'-bromo-5'-chloro-[1,1'-biphenyl]-4-carbonitrile (12.8 g, 43.8 mmol), phenanthren-9-ylboronic acid (9.7 g, 43.8 mmol), $Pd(PPh_3)_4$ (1 g, 0.87 mmol), and $K_2CO_3$ (12 g, 87 mmol). A mixture of deaerated toluene/THF/water (1.5:1.5:1, 175 mL) was added and the reaction mixture was stirred at reflux temperature under a nitrogen atmosphere for 23 h. After cooling down to ambient temperature, all volatiles were removed in vacuo and the resulting solid was dissolved in chloroform/water. The organic phase was washed with water three times, dried over $MgSO_4$ and filtered over a pad of silica. The filtrate was concentrated in vacuo to a minimal volume and n-hexane was added. The resulting white precipitate was isolated by suction filtration, washed with n-hexane and dried to afford 6.9 g (40%) of an off-white solid after drying. HPLC: 94.7%; GC-MS: m/z = 389.

**3",5"-bis(benzo[d]thiazol-2-yl)-5'-(phenanthren-9-yl)-[1,1':3',1"-terphenyl]-4-carbonitrile**

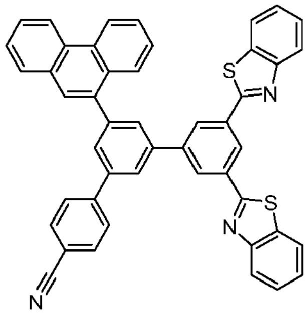

A flask was flushed with nitrogen and charged with 3'-chloro-5'-(phenanthren-9-yl)-[1,1'-biphenyl]-4-carbonitrile (4.8 g, 12.4 mmol), 2,2'-(5-(4,4,5,5-tetramethyl-1,3,2-dioxaborolan-2-yl)-1,3-phenylene)bis(benzo[d]thiazole) (7 g, 14.9 mmol), chloro(crotyl)(2-dicyclohexylphosphino-2',6'-dimethoxybiphenyl)palladium(II) (0.15 g, 0.25 mmol), and $K_3PO_4$ (5.3 g, 24.8 mmol). A mixture of deaerated THF/water (4:1, 100 mL) was added and the reaction mixture was stirred at 45 °C under a nitrogen atmosphere for 22 h. After cooling down to ambient temperature, the resulting precipitate was

isolated by suction filtration and washed with THF and water. The obtained solid was dissolved in dichloromethane and washed with water five times. After drying over $MgSO_4$, the organic phase was filtered over a pad of Florisil and silica. After rinsing with additional dichloromethane, the colorless filtrate was concentrated in vacuo to a minimal volume and n-hexane was added. The resulting white precipitate was isolated by suction filtration and washed with n-hexane. Further purification was achieved by trituration with hot acetonitrile and recrystallization from toluene to afford 2.5 g (29%) of a white solid after drying. Final purification was achieved by sublimation. HPLC: 99.2%; ESI-MS: m/z = 698 ($[M+H]^+$).

**Synthesis of inventive compound 7:**

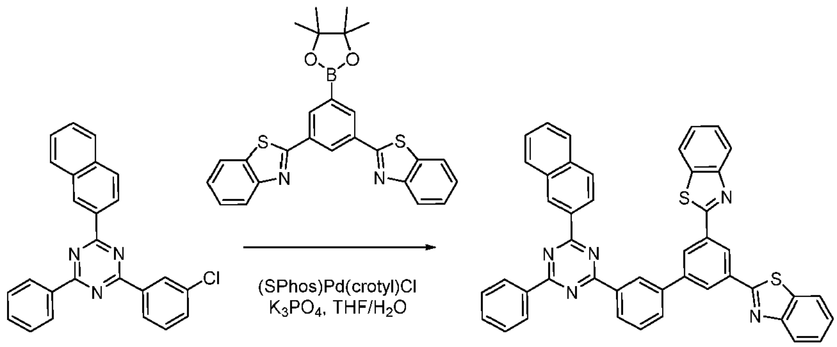


1891018-87-3

**2,2'-(3'-(4-(naphthalen-2-yl)-6-phenyl-1,3,5-triazin-2-yl)-[1,1'-biphenyl]-3,5-diyl)bis(benzo[d]thiazole)**

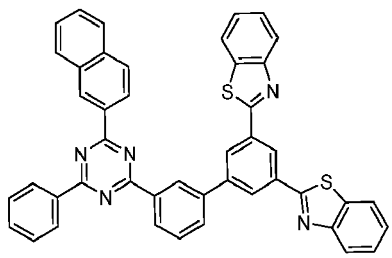

A flask was flushed with nitrogen and charged with 2-(3-chlorophenyl)-4-(naphthalen-2-yl)-6-phenyl-1,3,5-triazine (5.4 g, 13.8 mmol), 2,2'-(5-(4,4,5,5-tetramethyl-1,3,2-dioxaborolan-2-yl)-1,3-phenylene)bis(benzo[d]thiazole) (7.8 g, 16.6 mmol), chloro(crotyl)(2-dicyclohexylphosphino-2',6'-dimethoxybiphenyl)palladium(II) (0.17 g, 0.28 mmol), and $K_3PO_4$ (5.9 g, 27.6 mmol). A mixture of deaerated THF/water (4:1, 125 mL) was added and the reaction mixture was stirred at 45 °C under a nitrogen atmosphere for 18 h. Without cooling down, the precipitate was isolated by suction filtration and washed with THF. The obtained solid was dissolved in hot chlorobenzene and filtered over a pad of Florisil and silica. After rinsing with additional hot chlorobenzene, the filtrate was concentrated in vacuo and the resulting white precipitate was isolated by suction filtration and washed with chlorobenzene and dichloromethane to afford 7.2 g (74%) of a white solid after drying. Final purification was achieved by sublimation. HPLC: 99.1%; ESI-MS: m/z = 702 ($[M+H]^+$).

General procedure for fabrication of OLEDs

For top emission OLED devices, inventive examples and comparative examples, a glass substrate was cut to a size of 50 mm x 50 mm x 0.7 mm, ultrasonically cleaned with isopropyl alcohol for 5 minutes and then with pure water for 5 minutes, and cleaned again with UV ozone for 30 minutes, to prepare a first electrode. 100 nm Ag were deposited as anode at a pressure of $10^{-5}$ to $10^{-7}$ mbar to form the anode.

Then, 92 vol.-% Biphenyl-4-yl(9,9-diphenyl-9H-fluoren-2-yl)-[4-(9-phenyl-9H-carbazol-3-yl)phenyl]-amine (CAS 1242056-42-3) with 8 vol.-% 2,2',2"-(cyclopropane-1,2,3-triylidene)tris(2-(p-cyanotetrafluorophenyl)acetonitrile) was vacuum deposited on the ITO electrode, to form a HIL having a thickness of 10 nm. Then, Biphenyl-4-yl(9,9-diphenyl-9H-fluoren-2-yl)-[4-(9-phenyl-9H-carbazol-3-yl)phenyl]-amine was vacuum deposited on the HIL, to form a HTL having a thickness of 118 nm.

Then N,N-bis(4-(dibenzo[b,d]furan-4-yl)phenyl)-[1,1':4',1"-terphenyl]-4-amine (CAS 1198399-61-9) was vacuum deposited on the HTL, to form an electron blocking layer (EBL) having a thickness of 5 nm.

Then, the emission layer was deposited. For comparative example-1, example-1, example-2 and example-4 97 vol.-% 2-(10-phenyl-9-anthracenyl)-benzo[b]naphtho[2,3-d]furan (= H-1, CAS 1627916-48-6) as EML host and 3 vol.-% BD200 (Sun Fine Chemicals) as fluorescent blue dopant were deposited on the EBL, to form a blue-emitting EML with a thickness of 20 nm. For comparative example-2, comparative example-3, example 3, example 5, example 6, example 7 and example 8 97 vol.-% H09 (Fluorescent-blue host material, Sun Fine Chemicals) as EML host and 3 vol.-% BD200 (Sun Fine Chemicals) as fluorescent blue dopant were deposited on the EBL, to form a blue-emitting EML with a thickness of 20 nm.

Then, for top emission OLED devices of configuration A, the hole blocking layer was deposited on the emission layer with a thickness of 5 nm. For examples 1 to 5 compounds of formula 1 were deposited on the emission layer, according to table 6. For comparative examples-1 and -2 the comparative compound-1 was deposited on the emission layer as the hole blocking layer.

Then, for all devices of configuration A the electron transporting layer is formed on the hole blocking layer with a thickness of 31 nm by co-deposition of 2-([1,1'-biphenyl]-4-yl)-4-(9,9-diphenyl-9H-fluoren-4-yl)-6-phenyl-1,3,5-triazine (CAS 1801992-44-8) and lithium quinolate (LiQ) in a wt% ratio of 1:1.

For top emission OLED devices of configuration B 2,4-diphenyl-6-(4',5',6'-triphenyl-[1,1':2',1":3",1"':3"',1""-quinquephenyl]-3""-yl)-1,3,5-triazine (CAS 2032364-64-8) was deposited as the hole blocking layer with a thickness of 5 nm on the emission layer. For examples 6 to 8 compounds of formula 1 were co-deposited with lithium quinolate (LiQ), according to table 7, in a wt% ratio of 1:1 on the hole blocking layer to form the electron transporting layer with a thickness of 31 nm. For comparative example-3 the comparative compound-2 was co-deposited with lithium quinolate (LiQ) in a wt% ratio of 1:1 on the hole blocking layer to form the electron transporting layer with a thickness of 31 nm.

Then, for both top emission OLED devices of configuration A and B the electron injection layer is formed on the electron transporting layer by deposing Yb with a thickness of 2 nm.

Ag is evaporated at a rate of 0.01 to 1 Å/s at $10^{-7}$ mbar to form a cathode with a thickness of 11 nm.

A cap layer of Biphenyl-4-yl(9,9-diphenyl-9H-fluoren-2-yl)-[4-(9-phenyl-9H-carbazol-3-yl)phenyl]-amine is formed on the cathode with a thickness of 75 nm.

Overview of compounds used (non-inventive and non-comparative)

| IUPAC name | Reference |
|---|---|
| Biphenyl-4-yl(9,9-diphenyl-9H-fluoren-2-yl)-[4-(9-phenyl-9H-carbazol-3-yl)phenyl]-amine (CAS 1242056-42-3) | US2016322581 |
| 4,4',4"-((1E,1'E,1"E)-cyclopropane-1,2,3-triylidenetris (cyanomethanylylidene))tris(2,3,5,6-tetrafluorobenzonitrile) | US2008265216 |
| N,N-bis(4-(dibenzo[b,d]furan-4-yl)phenyl)-[1,1':4',1"-terphenyl]-4-amine (CAS 1198399-61-9) | JP2014096418 |
| H09 (Fluorescent-blue host material) | Commercially available from Sun Fine Chemicals, Inc, S. Korea |
| BD200 (Fluorescent-blue emitter material) | Commercially available from Sun Fine Chemicals, Inc, S. Korea |
| 2-(10-phenyl-9-anthracenyl)-benzo[b]naphtho[2,3-d]furan (CAS 1627916-48-6) = H-1 | EP2924029 |
| 2,4-diphenyl-6-(4',5',6'-triphenyl-[1,1':2',1":3",1"':3"',1""-quinquephenyl]-3""-yl)-1,3,5-triazine (CAS 2032364-64-8) | WO 2016171358 |
| 2-([1,1'-biphenyl]-4-yl)-4-(9,9-diphenyl-9H-fluoren-4-yl)-6-phenyl-1,3,5-triazine (CAS 1801992-44-8) | KR101537500 |
| 8-Hydroxyquinolinolato-lithium (850918-68-2) = Additive-1 = LiQ | WO2013079217 |
| IUPAC name | Reference |
| Biphenyl-4-yl(9,9-diphenyl-9H-fluoren-2-yl)-[4-(9-phenyl-9H-carbazol-3-yl)phenyl]-amine (CAS 1242056-42-3) | US2016322581 |

(continued)

| IUPAC name | Reference |
|---|---|
| 4,4',4"-((1E,1'E,1"E)-cyclopropane-1,2,3-triylidenetris (cyanomethanylylidene))tris(2,3,5,6-tetrafluorobenzonitrile) | US2008265216 |
| N,N-bis(4-(dibenzo[b,d]furan-4-yl)phenyl)-[1,1':4',1"-terphenyl]-4-amine (CAS 1198399-61-9) | JP2014096418 |
| H06 (Fluorescent-blue host material) | Commercially available from Sun Fine Chemicals, Inc, S. Korea |
| BD200 (Fluorescent-blue emitter material) | Commercially available from Sun Fine Chemicals, Inc, S. Korea |
| 2-([1,1'-biphenyl]-4-yl)-4-(9,9-diphenyl-9H-fluoren-4-yl)-6-phenyl-1,3,5-triazine (CAS 1801992-44-8) | KR101537500 |
| 8-Hydroxyquinolinolato-lithium (850918-68-2) ==LiQ | WO2013079217 |

The OLED stack is protected from ambient conditions by encapsulation of the device with a glass slide. Thereby, a cavity is formed, which includes a getter material for further protection.

To assess the performance of the inventive examples compared to the existing art, the light output of the top emission OLEDs is measured under ambient conditions (20°C). Current voltage measurements are performed using a Keithley 2400 sourcemeter, and recorded in V at 10 mA/cm$^2$ for top emission devices, a spectrometer CAS140 CT from Instrument Systems, which has been calibrated by Deutsche Akkreditierungsstelle (DAkkS), is used for measurement of CIE coordinates and brightness in Candela. The current efficiency Ceff is determined at 10 mA/cm2 in cd/A.

In top emission devices, the emission is forward-directed, non-Lambertian and also highly dependent on the micro-cavity. Therefore, the external quantum efficiency (EQE) and power efficiency in lm/W will be higher compared to bottom emission devices.

## ***Technical Effect of the invention***

### Material property

The Tg of compounds of formula 1 (Table 4) are increased versus the comparative compound-1 (Table 3). The values are in a range suitable for use in organic electronic devices. Higher Tg values of materials used in organic electronics are generally preferred for device durability and robustness.

Table 5 shows that the LUMO energy levels and the dipole moments of compounds of formula 1 are in a range suitable for use as hole blocking materials or electron transporting materials in organic electronic devices.

### Top emission devices

Surprisingly, the operating voltage of top emission OLED devices is reduced when using compounds of formula 1 mixed with the additive LiQ as an electron transport layer. Also, the operating voltage of top emission OLED devices is reduced when using compounds of formula 1 as a hole blocking layer.

Surprisingly, the cd/A current efficiencies were increased when using compounds of formula 1 as a hole blocking layer.

Table 6 shows the operating voltage and cd/A efficiencies of top emission OLED devices comprising a hole blocking layer comprising a compound of formula 1. Table 7 shows the operating voltage of top emission OLED devices comprising an electron transport layer comprising a 1:1 wt% mixture of a compound of formula 1 and LiQ.

In summary, improved performance of top emission OLED devices can be achieved by using compounds of formula 1.

### Experimental data (overview)

**Table 3: Properties of comparative compounds**

| Compound name | Structure | Tg (°C) | Tm (°C) | TRO (°C) |
|---|---|---|---|---|
| Comparative compound-1 CAS 2032364-64-8 | 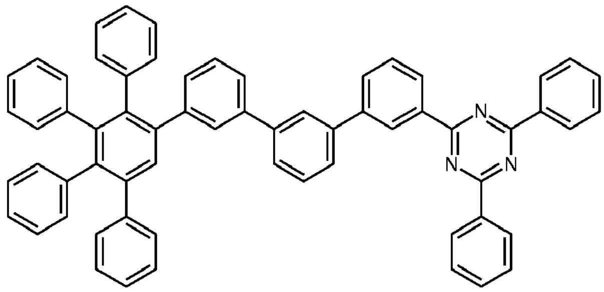 | 140 | - | 267 |
| Comparative compound-2 CAS 2032421-35-3 | 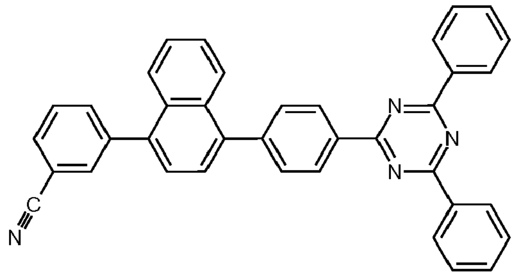 | 99 | 252 | 216 |

**Table 4: Properties of compounds of formula 1**

| Compound name | Structure | Tg (°C) | Tm (°C) | TRO (°C) |
|---|---|---|---|---|
| Inv-1 | 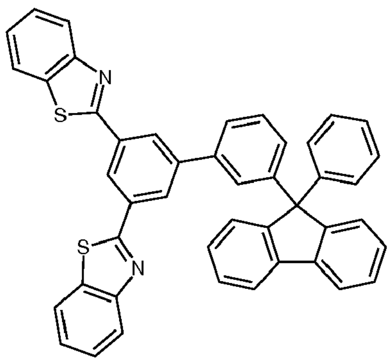 | 126 | 242 | 236 |
| Inv-2 | 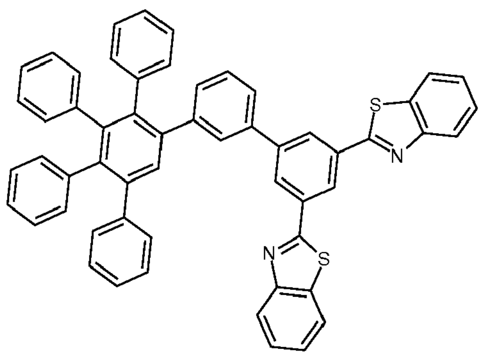 | 151 | 277 | 254 |

(continued)

| Compound name | Structure | Tg (°C) | Tm (°C) | TRO (°C) |
|---|---|---|---|---|
| Inv-3 | 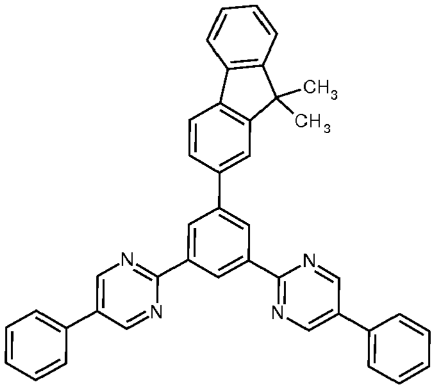 | 127 | 301 | 249 |
| Inv-4 | 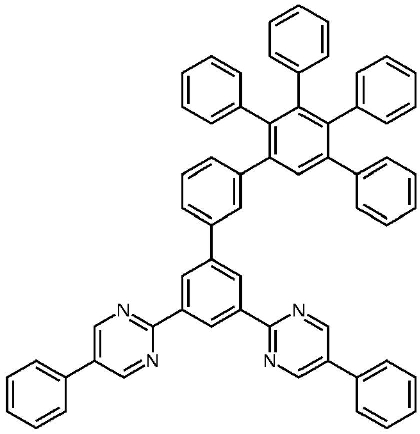 | 163 | 332 | 299 |
| Inv-5 | 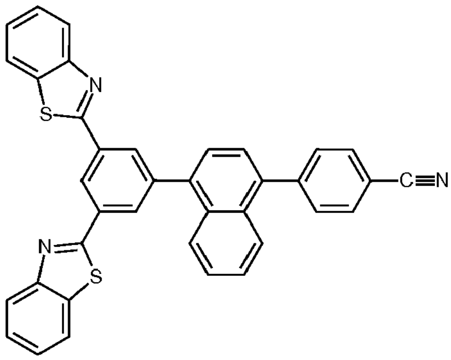 | 126 | 267 | 248 |

(continued)

| Compound name | Structure | Tg (°C) | Tm (°C) | TRO (°C) |
|---|---|---|---|---|
| Inv-6 | 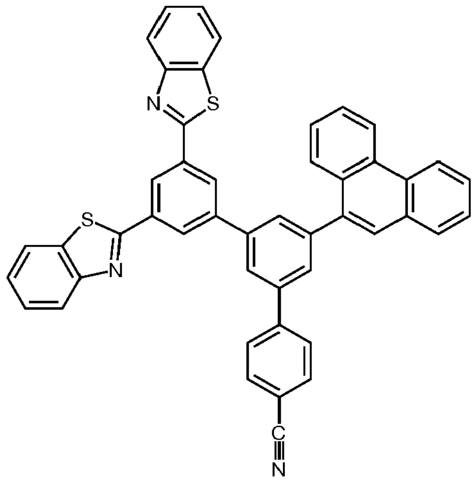 | 159 | 323 | 309 |
| Inv-7 | 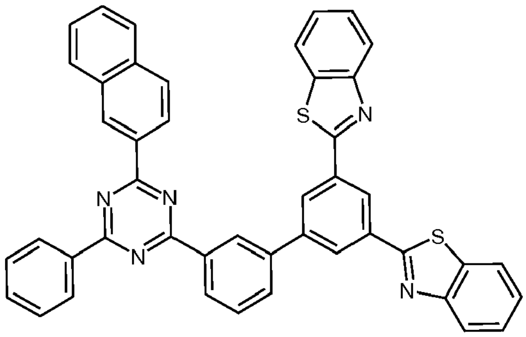 | 113 | 304 | 300 |

**Table 5: Energy levels and dipole moments of comparative compound and compounds of formula 1**

| Compound name | HOMO (eV) * | LUMO (eV) * | Dipole moment (Debye) * |
|---|---|---|---|
| Comparative compound-1 | -5.72 | -1.82 | 0.30 |
| Inv-1 | -5.80 | -1.82 | 0.56 |
| Inv-2 | -5.83 | -1.80 | 1.10 |
| Inv-3 | -5.44 | -1.65 | 2.29 |
| Inv-4 | -5.67 | -1.63 | 1.98 |
| Inv-5 | -5.93 | -1.97 | 5.35 |
| Inv-6 | -5.82 | -1.98 | 5.14 |
| Inv-7 | -5.92 | -1.92 | 0.64 |
| * Values calculated with B3LYP_Gaussian / 6-31G*, gas phase | | | |

**Table 6: Operating voltage of top emission organic electroluminescent devices comprising a compound of formula 1 in the hole blocking layer (configuration A).**

| | Material of Hole blocking layer | Thickness hole blocking layer (nm) | CIEy | Operating voltage (V) at 10 mA/cm$^2$ | Ceff (cd/A) at 10 mA/cm$^2$ |
|---|---|---|---|---|---|
| Comparative example 1 ● | Comparative compound 1 | 31 | 0.043 | 3.28 | 7.8 |
| Comparative example 2 + | Comparative compound 1 | 31 | 0.044 | 3.44 | 7.7 |
| Example 1 ● | Inv-1 | 31 | 0.046 | **3.22** | **8.3** |
| Example 2 ● | Inv-2 | 31 | 0.046 | **3.21** | **8.3** |
| Example 3 + | Inv-2 | 31 | 0.045 | **3.42** | **8.1** |
| Example 4 ● | Inv-3 | 31 | 0.046 | **3.24** | **8.4** |
| Example 5 + | Inv-4 | 31 | 0.048 | **3.61** | **9.2** |
| ● Comparative example 1, example 1, example 2 and example 4 were prepared using H-1 as the emitter host compound.<br>+ Comparative example 2, example 3 and example 5 were prepared using H09 as the emitter host compound. | | | | | |

**Table 7: Operating voltage of top emission organic electroluminescent devices comprising a 1:1 wt% mixture of compound of formula 1 with LiQ in the electron transport layer (configuration B).**

| | Material of Electron transport layer | Thickness electron transport layer (nm) | CIEy | Operating voltage (V) at 10 mA/cm$^2$ | Ceff (cd/A) at 10 mA/cm$^2$ |
|---|---|---|---|---|---|
| Comparative example 3 | Comparative compound 2:LiQ | 31 | 0.044 | 3.58 | 7.5 |
| Example 6 | Inv-5:LiQ | 31 | 0.046 | **3.45** | **7.8** |
| Example 7 | Inv-6:LiQ | 31 | 0.044 | **3.53** | **7.6** |
| Example 8 | Inv-7:LiQ | 31 | 0.048 | **3.46** | **7.8** |

From the Table 6 and 7 it can be clearly seen that the inventive compounds allow to build optoelectronic devices with an improved combination of operating voltage and efficiency.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims and the equivalents thereto. Furthermore, reference signs used in the description and claims do not limit the scope of the invention as claimed.

Means and methods:

Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment can be determined by a semi-empirical molecular orbital method.

In the context of the present invention, the dipole moment of a molecule especially means and /or includes that the dipole moment is calculated where geometries of the corresponding molecular structures are optimized using the hybrid functional B3LYP with the 6-31G* basis set as implemented in the program package TURBOMOLE V6.5. If more than one conformation is viable, the conformation with the lowest total energy is selected to determine the bond lengths of the molecules. The dipole moment then can be calculated with said B3LYP_Gaussian / 6-31G* program for the gas phase

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

Reduction potential

The reduction potential is determined by cyclic voltammetry with potenioststic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard $Fc^+/Fc$ redox couple, afforded finally the values reported above. All studied compounds as well as the reported comparative compounds showed well-defined reversible electrochemical behaviour.

HOMO and LUMO energy levels

The energies of the highest occupied molecular orbital, also named HOMO, and of the lowest unoccupied molecular orbital, also named LUMO, are measured in electron volt (eV) indirectly by cyclic voltammetry vs ferrocene or can be calculated Such DFT calculations may be carried out using the program package TURBOMOLE V6.5 (Provider: TURBOMOLE GmbH, Litzenhardtstrasse 19, 76135 Karlsruhe, Germany). The optimized geometries and the HOMO/LUMO energy levels of the molecular structures are determined using the hybrid functional B3LYP with a 6-31G* basis set. If more than one conformation is viable, the conformation with the lowest total energy is selected.

The terms "OLED" and "organic light-emitting diode" are simultaneously used and have the same meaning. The term "organic electroluminescent device" as used herein may comprise both organic light emitting diodes as well as organic light emitting transistors (OLETs).

## Claims

1. A compound for an electroluminescent device having the structure (I):

$$(Het)_2\text{-}Ar^1\text{-}[L(Ar^2)_m(R^1)_n(R^2)_p]_q \qquad (I)$$

wherein

Het is an aryl or heteroaryl substituted or unsubstituted $C_2$ to $C_{30}$ heteroaryl group, whereby Het is not carbazoyl nor triazolyl,
$Ar^1$ is a $C_6$ to $C_{30}$ aryl group or $C_2$ to $C_{30}$ heteroaryl group, whereby $Ar^1$ is not triazine,
L is a $C_6$ to $C_{30}$ substituted or unsubstituted aryl group
$Ar^2$ is a substituted, aryl or heteroaryl or $R^1$ substituted or unsubstituted $C_6$ to $C_{42}$ aryl group or $C_2$ to $C_{42}$ heteroaryl group
$R^1$ is a polar uncharged organic moiety
$R^2$ is selected from the group comprising aryl or heteroaryl- substituted vinyl, -O-, -S-, -NR'-, alkylene, arylene.
m to p are independently an integer of 0 to 5
q is 1, 2 or 3, whereby when q > 1 then any moieties L can be different from each other and two suitable moieties L may form a macrocyclus with $Ar^1$ and $R^2$;

whereby when Het is triphenylsubstituted imidazole and L is anthracenyl or when Het is benzimidazole then $Ar^1$ is not carbazolyl

2. The compound of Claim 1, whereby $Ar^1$ is selected from phenyl, pyridyl, carbazolyl and fluorenyl

3. The compound of Claim 1 or 2, whereby L is selected from phenyl, naphtanyl, anthracenyl and fluorenyl

4. The compound of any of the Claims 1 to 3, whereby Het is a substituted or unsubstituted bicyclic aryl moiety comprising a heteroaryl-containing five-membered ring.

5. The compound of any of the claims 1 to 3, whereby Het is a phenyl substituted six-membered heteroaryl.

6. The compound of any of the claims 1 to 5, whereby Het is selected from

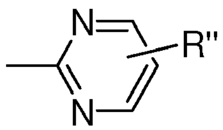 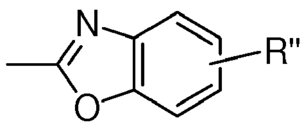 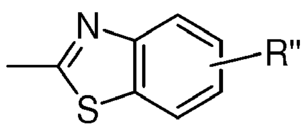 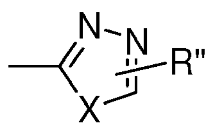

whereby X = N or S and R'' is hydrogen, phenyl, naphthyl.

7. The compound of any of the claims 1 to 6, whereby the moiety Het-Rg-Het, whereby
Rg= $Ar^1$ when $Ar^1$ is monocyclic or the two moieties Het are bound to two different rings; or
Rg= the aromatic ring within $Ar^1$ to which both Het are bound
has a $C_{2v}$ - symmetry.

8. The compound of any of the claims 1 to 7, whereby m+n+p >0.

9. The compound of any of the claims 1 to 8, whereby $1 \leq m+n+p \leq 5$

10. The compound of any of the claims 1 to 9, whereby $R^1$ is selected from substituted or unsubstituted $C_2$ to $C_{30}$ heteroaryl group, CN and phosphine oxide

11. An electronic device comprising a first electrode, a second electrode, and arranged between the first and second electrode, a layer comprising a compound according to any of the preceding claims 1 to 10.

12. The electronic device of claim 11, whereby the electronic device comprises a hole blocking layer comprising a

compound according to any of the preceding claims 1 to 10.

**13.** The electronic device of claim 11 or 12, whereby the electronic device comprises an electron transport layer comprising a compound according to any of the preceding claims 1 to 10

**14.** The electronic device according to any of the claims 11 to 13, wherein the electronic device is an electroluminescent device, preferably an organic light emitting diode.

**15.** A display device comprising an electronic device according to any of the preceding claims 11 to 13, preferably, the display device comprises an organic light emitting diode according to claim 14.

Fig. 1

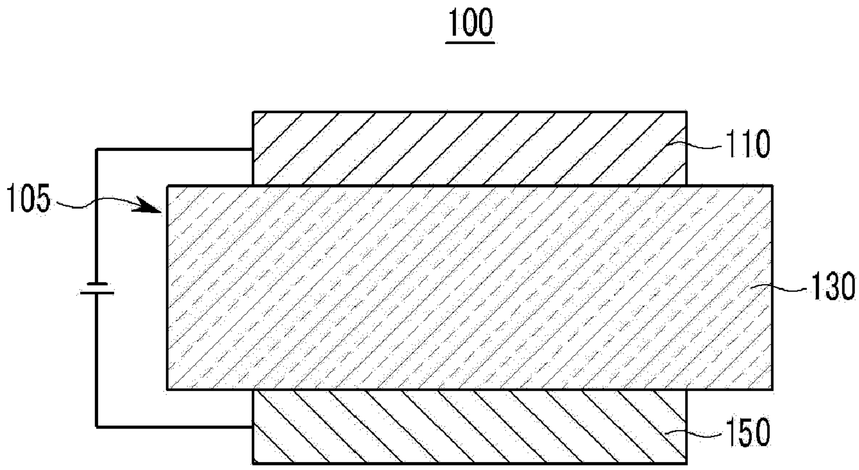


Fig. 2

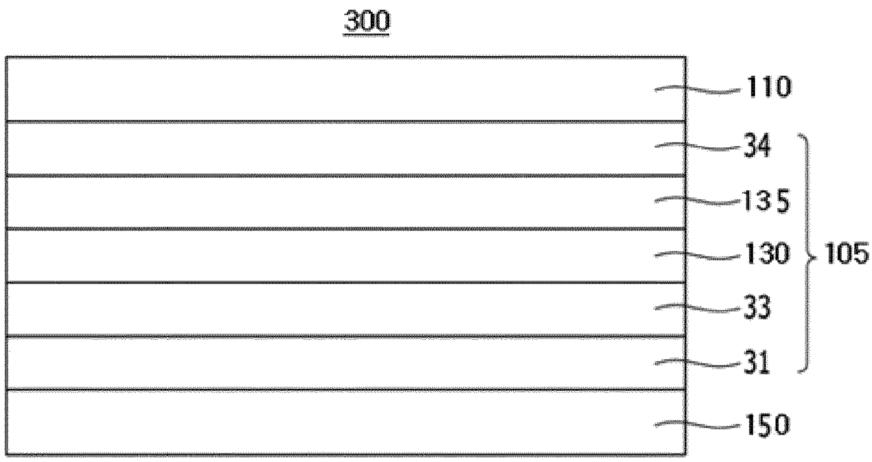

Fig. 3

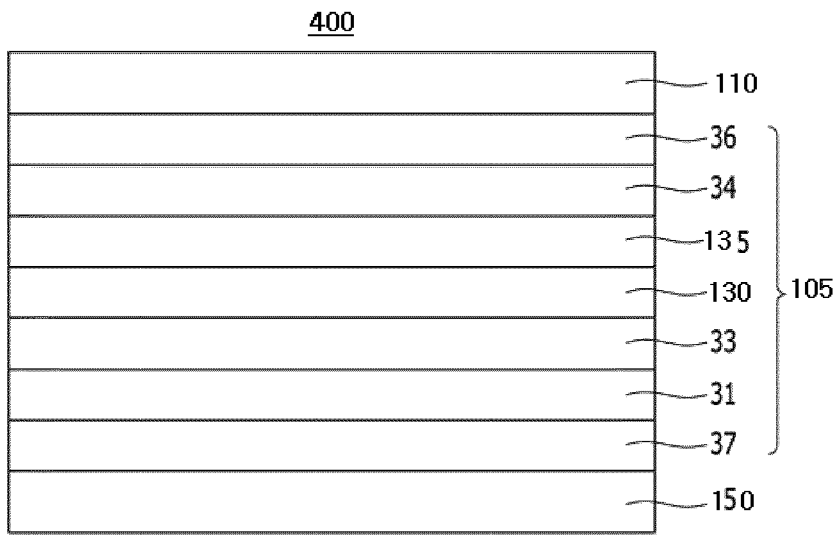

Europäisches Patentamt
European Patent Office
Office européen des brevets

# EUROPEAN SEARCH REPORT

Application Number
EP 18 15 9060

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2017 0092131 A (LG CHEMICAL LTD [KR]) 10 August 2017 (2017-08-10) * abstract * * examples; page 4 - page 6 * * claim 6 * | 1-15 | INV. C07D239/26 C07F9/53 C07D417/14 C07D471/04 C07D263/57 C07D271/107 C07D277/22 C07D277/66 C07D513/04 C07D285/12 C07F9/6512 C07F9/653 C07F9/6539 C07F9/6541 C07F9/6561 |
| X | US 7 282 586 B1 (YEN FENG-WEN [TW] ET AL) 16 October 2007 (2007-10-16) * abstract * * column 7 - column 18; compounds 1-6 * * claim 1 * | 1-15 | |
| X | CN 103 508 940 B (KUNSHAN VISIONOX DISPLAY TECH; UNIV TSINGHUA; BEIJING VISIONOX TECHNOL) 3 May 2017 (2017-05-03) * abstract * * page 26 - page 39; examples TM1-TM20, TM33-TM40, TM45-TM48 * * claim 1 * | 1-15 | |
| X | CN 104 193 738 B (SHANGHAI TAOE CHEMICAL TECHNOLOGY CO LTD) 18 May 2016 (2016-05-18) * abstract * * page 9 - page 10; compounds 1-24 * * claim 1 * | 1-15 | TECHNICAL FIELDS SEARCHED (IPC) C07D C07F |
| X | WO 2012/161382 A1 (CHEIL IND INC [KR]; YU EUN-SUN [KR]; CHAE MI-YOUNG [KR]; KANG EUI-SU [] 29 November 2012 (2012-11-29) * abstract * * compounds: A2-A6, A8, A9, A11-A15, A17, A18, A20-A27, A29-A33, A32-A37, B2-B5, B7-B10, B15, B16, B18-B22, B24, B25; page 5 * * claim 1 * | 1-7,9-15 | |

The present search report has been drawn up for all claims

| Place of search | Date of completion of the search | Examiner |
|---|---|---|
| Munich | 16 October 2018 | Bissmire, Stewart |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 15 9060

This annex lists the patent family members relating to the patent documents cited in the above-mentioned European search report. The members are as contained in the European Patent Office EDP file on
The European Patent Office is in no way liable for these particulars which are merely given for the purpose of information.

16-10-2018

| Patent document cited in search report | Publication date | Patent family member(s) | Publication date |
|---|---|---|---|
| KR 20170092131 A | 10-08-2017 | NONE | |
| US 7282586 B1 | 16-10-2007 | NONE | |
| CN 103508940 B | 03-05-2017 | NONE | |
| CN 104193738 B | 18-05-2016 | NONE | |
| WO 2012161382 A1 | 29-11-2012 | CN 103443241 A | 11-12-2013 |
| | | EP 2663609 A1 | 20-11-2013 |
| | | KR 20120131870 A | 05-12-2012 |
| | | TW 201247645 A | 01-12-2012 |
| | | US 2014027750 A1 | 30-01-2014 |
| | | WO 2012161382 A1 | 29-11-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2005086251 A
- EP 1837926 B1
- WO 2007107306 A
- WO 2007107356 A
- US 2016322581 A
- US 2008265216 A
- JP 2014096418 B
- EP 2924029 A
- WO 2016171358 A
- KR 101537500
- WO 2013079217 A

### Non-patent literature cited in the description

- **N. CHANDRASHEKHAR et al.** *Magn. Reson. Chem.,* 2008, vol. 46, 769-774
- *CHEMICAL ABSTRACTS,* 1242056-42-3
- *CHEMICAL ABSTRACTS,* 1198399-61-9
- *CHEMICAL ABSTRACTS,* 1627916-48-6
- *CHEMICAL ABSTRACTS,* 1801992-44-8
- *CHEMICAL ABSTRACTS,* 2032364-64-8
- *CHEMICAL ABSTRACTS,* 2032421-35-3